# EUROPEAN PATENT SPECIFICATION

(11) **EP 3 096 713 B1**
(45) Date of publication and mention of the grant of the patent: **21.09.2022**
(21) Application number: 15740450.0
(22) Date of filing: 23.01.2015
(51) Int. Cl.: A61F 2/24

(54) **ENGINEERED POLYMERIC VALVES, TUBULAR STRUCTURES AND SHEETS**
MANIPULIERTE POLYMERE VENTILE, ROHRFÖRMIGE STRUKTUREN UND FOLIEN
VALVULES POLYMÈRES TECHNIQUES, STRUCTURES TUBULAIRES ET FEUILLES

(30) Priority: 23.01.2014 US 201461930746 P
(43) Date of publication of application: 30.11.2016
(73) Proprietor: President and Fellows of Harvard College, Cambridge, MA 02138 (US)
(72) Inventor: PARKER, Kevin Kit, Cambridge, MA 02138 (US); CAPULLI, Andrew K., Billerica, MA 01862 (US); GOSS, Josue A., Cambridge, MA 02138 (US)
(74) Representative: Lock, Graham James
(86) International application number: PCT/US2015/012646
(87) International publication number: WO 2015/112839

(56) References cited:
- WO-A1-2010/041944
- WO-A1-2013/018021
- NL-C2- 1 008 349
- US-A1- 2004 034 408
- US-A1- 2008 038 352
- US-A1- 2008 109 070
- US-A1- 2008 131 965
- US-A1- 2012 296 418
- US-A1- 2013 312 638
- US-A1- 2013 325 117
- US-B2- 7 981 353

## Description

### BACKGROUND OF THE INVENTION

Over 150,000 surgical procedures are performed each year to replace damaged or diseased cardiac valves worldwide. For example, mechanical heart valve prostheses can be used to replace any naturally occurring cardiac valves. Operating much like a rigid mechanical check valve, mechanical heart valves are robust and long-lived. However, mechanical valves suffer from the disadvantage that they are thrombogenic and, thus, the patient requires lifetime anticoagulant therapy which is expensive and potentially dangerous in that it may cause abnormal bleeding which, in itself, can cause a stroke if the bleeding occurs within the brain. In addition, mechanical valves also generate a clicking noise when the mechanical closure seats against the associated valve structure at each beat of the heart.

One alternative to mechanical valves is tissue-type or "bioprosthetic" valves. Bioprosthetic valves are generally made from naturally-derived xenogeneic tissues fixed with glutaraldehyde-based processes. Bioprosthetic valves are constructed either by sewing pig aortic valves to a stent to hold the leaflets in proper position, or by constructing valve leaflets using pericardial sac, such as bovine-derived pericardium, and sewing the leaflets to a stent. The stents can be rigid or slightly flexible and are covered with cloth, usually a synthetic material. The major disadvantage of bioprosthetic valves is that they lack the long-term durability of mechanical valves. In addition, naturally occurring processes within the human body can attack and stiffen or "calcify" the tissue leaflets of the bioprosthetic valve over time, particularly at high-stress areas of the valve, such as at the commissure junctions between the valve leaflets and at the peripheral leaflet attachment points or "cusps" at the outer edge of each leaflet. Further, bioprosthetic valves are subject to stresses from constant mechanical operation within the body.

Another alternative is tissue-engineered heart valves which have been proposed by physicians and scientists alike to be the ultimate solution for treating valvular heart disease. Rather than replacing a diseased or defective native valve with a mechanical or animal tissue-derived artificial valve, a tissue-engineered valve is a living organ, able to respond to growth and physiological forces in the same way that the native valve does. In particular, a whole porcine aortic valve that has been previously cleaned of all pig cells is implanted in a subject leaving an intact, mechanically sound connective tissue matrix. The cells of the patients are expected to repopulate and revitalize the acellular matrix, creating living tissue that already has the complex microstructure necessary for proper function and durability. Although these tissue-engineered valves can overcome the need for anticoagulants as well as the need for open heart surgery, their very composition sacrifices durability. On average, these heart valves are only expected to last 15 years in the patient before the over 600 million open-close cycles within this time frame cause significant fatigue that warrants replacement. Although this lifecycle of these tissue-engineered valves may be less relevant for the aging patient population that requires replacement semilunar valves as a result of atherosclerosis or calcification, it is a significant problem for children born with congenital valve defects. One out of every 110 babies born in the US suffers from congenital heart disease, with valve malformation among the most common abnormalities. Because these current animal sourced valves are non-regenerative and eventually fatigue, children needing valve repair or replacement will have to undergo numerous surgeries throughout their lifetime. In addition, current tissue-engineered valves consist of foreign body material.

In order to overcome the limitations of tissue-engineered valves, there has been a focus on replacing the fixed-tissue basis of the valve with a degradable scaffold material that is seeded with stem cells in the hopes of promoting endogenous repair and remodeling once implanted. Although many of these studies have shown promising results of tissue remodeling and integration into the body, there still remains a significant gap between research usage and commercial adoption of this type of tissue engineered valve. Typical scaffolding materials are either polymers composed of chemical substances like poly-glycolic acid, poly-4-hydroxybutyrate, polyhydroxyalkanoate and gels out of extracellular matrix proteins such as collagen or fibrin. Unfortunately, these materials are still far from ideal. They are expensive, potentially immunogenic and further they show toxic degradation and inflammatory reactions. In addition, they are of poor resorbability. As a consequence, there is a lack of growth and risk of thromboembolic complications, degeneration and infections. Furthermore, tissue engineering of highly customized valve scaffolds doped with growth factors or seeded with stem cells and cultured in the lab prior to implantation results in a very costly and specialized valve implant.

Prosthetic engineered tissue valves have recently been fabricated using polymeric nanofibers (see, *e.g.* PCT/US2013/051267, filed July 19, 2013, and U.S. Patent Application No. 14/415,945, filed January 20, 2015. These valves, although mechanically sound and biocompatible require several fabrication steps. The separate fabrication steps of the valve leaflets and conduit require the valve leaflets and conduit to be connected *via* suture, adhesive, heat-weld, or some other fixation technique, such as suturing, at connection points. The connection points are high load bearing points of the device and are also points where the device has been weakened by forming the connection. As such, there may be an increased risk of valve failure at the connection points and/or lack of tissue remodeling as native cells fail to repopulate properly at the connection points. Furthermore, to deliver these valves *via* a minimally invasive procedure, pieces of the valve (e.g., valve leaflets) may need to be cut and sutured onto a stent by a skilled surgeon, which increases the complexity and time required for the procedure.

Accordingly, there is a need in the art for improved valves that are durable and can mimic the function of the human cardiac valves and do not have the side-effects associated with current devices.

WO2010/041944 relates to a multilayer preform obtained by electro-spinning as well as a method for producing the preform and the use thereof. The document discloses a multilayer preform obtained by electro-spinning, which is suitable as a scaffold for a prosthesis. The preform comprises at least one layer of micro fibres and at least one layer of nanofibres, wherein the pore size of the at least one layer of microfibres is in the range of 1 - 300 micrometre and in that the pore size of the at least one layer of nanofibres is in the range of 1 - 300 micrometre. The document describes the use of the preform as a substrate for growing human or animal tissue.

### SUMMARY OF THE INVENTION

The present invention is based, at least in part, on the discovery of methods for the assembly of engineered tubular structures, such as valves, comprising oriented polymeric fibers, *e.g*., nanofibers. The methods of the present invention are simpler than previous methods and permit the fabrication of tubular structures, such as replacement valves, without the use of sutures to attach valve leaflets to a valve conduit. In addition, the methods of the present invention permit the fabrication of valves without the use of melt processing, thereby substantially eliminating failure at high-stress points.

In one aspect, the present invention provides an engineered valve, comprising:
a tubular wall comprising micron, submicron or nanometer dimension polymer fibers defining a shape of the tubular wall, the tubular wall having an inner surface, a first portion, and a second portion;
one or more leaflets extending from, and integral with, the inner surface of the tubular wall, the one or more leaflets each comprising micron, submicron or nanometer dimension polymer fibers defining the shape of the corresponding leaflet;
wherein the one or more leaflets are configured for one-way fluid flow through the first portion of the tubular wall, past the one or more leaflets, and into the second portion of the tubular wall, and wherein the second portion of the tubular wall includes all of the tubular wall downstream of the one or more leaflets;
wherein, for each of the one or more leaflets, at least some of the micron, submicron or nanometer dimension polymer fibers forming a part of the leaflet interpenetrate with at least some of the micron, submicron or nanometer dimension polymer fibers forming a part of the first portion of the tubular wall; and
wherein the micron, submicron or nanometer dimension polymer fibers of the tubular wall and the micron, submicron or nanometer dimension polymer fibers of the one or more leaflets are configured to form a polymeric fiber scaffold for cellular ingrowth into the tubular wall and the one or more leaflets.

The engineered valve includes a tubular wall including micron, submicron or nanometer dimension polymer fibers defining a shape of the tubular wall, the tubular wall having an inner surface. The engineered valve also includes one or more leaflets extending from, and integral with, the inner surface of the tubular wall, the one or more leaflets including micron, submicron or nanometer dimension polymer fibers defining the shape of the one or more leaflets. The engineered valve may be referred to as a suture-free valve because no sutures or other attachment means are used to attach leaflets to the tubular wall of the valve.

In a second aspect, the invention provides a method of making a valve including a tubular wall and one or more leaflets integral with and extending from an inner surface of the tubular wall, the method comprising:
collecting a first portion of formed polymeric fibers on an outer surface of a first mandrel to at least partially form a first portion of a tubular wall and one or more leaflets connected to, and integral with, an inner surface of the first portion of the tubular wall, the formed polymeric fibers including micron, submicron, and/or nanometer dimension polymeric fibers, wherein, for each of the one or more leaflets, at least some of the micron, submicron or nanometer dimension polymer fibers forming a part of the leaflet interpenetrate with at least some of the micron, submicron or nanometer dimension polymer fibers forming a part of the upstream first portion of the tubular wall, the outer surface of the first mandrel including:
   a first tubular forming portion having a shape corresponding to the inner surface of the first portion of the tubular wall, and
   one or more leaflet forming portions, each having a shape corresponding to a first surface of a corresponding one of the one or more leaflets of the resulting valve;
   positioning a second mandrel with respect to the first mandrel having the collected first portion of polymer fibers thereon, the second mandrel having an outer surface including a second tubular forming portion having a shape corresponding to an inner surface of a second portion of the resulting tubular wall, the first mandrel and second mandrel together forming a combined mandrel; and
collecting a second portion of the formed polymeric fibers on an outside surface of the combined mandrel thereby forming, at least, the second portion of the tubular wall, wherein the second portion of the resulting tubular wall is the portion of the resulting tubular wall that is downstream of the one or more leaflet during one-way fluid flow through the resulting valve;
wherein the micron, submicron or nanometer dimension polymer fibers of the tubular wall and the micron, submicron or nanometer dimension polymer fibers of the one or more leaflets are configured to form a polymeric fiber scaffold for cellular ingrowth into the tubular wall and the one or more leaflets.

In some embodiments at least some of the micron, submicron or nanometer dimension polymer fibers of the tubular wall interpenetrate with at least some of the micron, submicron or nanometer dimension polymer fibers of the one or more leaflets.

In some embodiments the tubular wall of the engineered valve further includes a stent embedded in the micron, submicron or nanometer dimension polymer fibers.

In some embodiments, the engineered valve is formed by the methods described herein.

In some embodiments, the micron, submicron or nanometer dimension polymer fibers of the tubular wall and the micron, submicron or nanometer dimension polymer fibers of the one or more leaflets are configured to form a polymeric fiber scaffold for cellular ingrowth.

An embodiment is a method of making an engineered tubular structure. The method includes forming a first portion of polymeric fibers by ejecting or flinging a polymer from a reservoir onto a first mandrel, the polymeric fibers including micron, submicron, and/or nanometer dimension polymeric fibers. The method also includes collecting the first portion of formed polymeric fibers on an outside surface of a mandrel. The method further includes positioning a stent over the first portion of formed polymeric fibers deposited on the outside surface of the mandrel and forming a second portion of the polymeric fibers by ejecting or flinging the polymer from the reservoir. The method also includes collecting the second portion of the polymeric fibers on an outside surface of the stent, thereby forming a tubular wall with the stent embedded therein.

A method, not forming part of the invention, for treating a subject having a defective or weakened cardiac valve is described herein. The method includes providing an engineered valve which includes a tubular wall including micron, submicron or nanometer dimension polymer fibers defining a shape of the tubular wall, the tubular wall having an inner surface. The engineered valve also includes one or more leaflets extending from, and integral with, the inner surface of the tubular wall, the one or more leaflets including micron, submicron or nanometer dimension polymer fibers defining the shape of the one or more leaflets. The method further includes replacing the weakened or defective valve in the subject with the engineered valve, thereby treating the subject. As described herein, the weakened or defective valve of the subject is replaced with an engineered valve that is not seeded with cells prior to being placed in the subject, *e.g.,* the native cells of the subject populate the valve to, *e.g.,* form a functional tissue. As described herein, the weakened or defective valve of the subject is replaced with an engineered valve that is seeded with cells (*e.g*., autologous cells), and optionally cultured under appropriate conditions, prior to being placed in the subject.

A method of making a valve including a tubular wall and one or more leaflets integral with and extending from an inner surface of the tubular wall is described herein. The method includes collecting a first portion of formed polymeric fibers on an outer surface of a first mandrel to at least partially form a first portion of a tubular wall and one or more leaflets connected to, and integral with, an inner surface of the first portion of the tubular wall. The formed polymeric fibers include micron, submicron, and/or nanometer dimension polymeric fibers. The first mandrel has an outside surface including a first tubular forming portion having a shape corresponding to the inner surface of the first portion of the tubular wall, and one or more leaflet forming portions. Each leaflet forming portion has a shape corresponding to a first surface of a corresponding one of the one or more leaflets of the resulting valve. The method also includes positioning a second mandrel with respect to the first mandrel having the collected first portion of polymer fibers thereon. The second mandrel has an outer surface including a second tubular forming portion with a shape corresponding to an inner surface of a second portion of the resulting tubular wall. The first mandrel and second mandrel together form a combined mandrel. The method also includes collecting a second portion of the polymeric fibers on an outside surface of the combined mandrel thereby forming, at least, the second portion of the tubular wall.

In some embodiments the method of the invention further includes forming the first portion of the polymeric fibers by ejecting or flinging a polymer from a reservoir onto the first mandrel, and forming the second portion of the polymeric fibers by ejecting or flinging the polymer from the reservoir onto the combined mandrel. In some embodiments collecting the first portion of the polymeric fibers includes rotating the first mandrel about a mandrel rotation axis in a path of the ejected or flung polymer fibers, and collecting the second portion of the polymeric fibers includes rotating the combined mandrel about the mandrel rotation axis in a path of the ejected or flung polymer fibers. In some embodiments the polymer is ejected from a reservoir rotating about an deposition rotation axis or flung from a structure rotating about the deposition rotation axis where the deposition rotation axis forms an angle of between 0 and 180 with the mandrel rotation axis during collection of the first portion of the polymeric fibers and during collection of the second portion of the polymeric fibers.

In some embodiments a surface of the second mandrel includes one or more leaflet forming portions, each having a shape corresponding to a second surface of a corresponding one of the one or more leaflets in the resulting valve. In some embodiments at least one first leaflet forming portion of the first mandrel has a concave shape, and at least one leaflet portion of the second mandrel has a convex shape.

In some embodiments the first portion of the polymeric fibers is disposed between the first mandrel and the second mandrel and on the first tubular forming portion of the outside surface of the first mandrel when the second mandrel is positioned with respect to the first mandrel to form the combined mandrel. In some embodiments the combined mandrel forms a crevasse between the first mandrel and the second mandrel with the crevasse extending radially inward from the outer surface of the combined mandrel. In some embodiments the first portion of the polymeric fibers is disposed within the crevasse and extends at least to a boundary between the crevasse and first tubular forming portion, such that when the second portion of the polymeric fibers is collected on the combined mandrel, the second portion of polymeric fibers integrates with the first portion of polymeric fibers.

In some embodiments positioning the second mandrel with respect to the first mandrel includes engaging at least one first engagement portion of the first mandrel with a corresponding at least one second engagement portion of the second mandrel. In some embodiments the at least one first engagement portion includes at least one protrusion, and the at least one second engagement portion includes at least one recess configured to receive the at least one protrusion.

In some embodiments the method further includes positioning a stent over the second portion of the polymeric fibers collected on the outside surface of the combined mandrel and collecting a third portion of the polymeric fibers on an outside surface of the stent to form the tubular wall with the stent embedded therein. In some embodiments in which the stent is embedded in the tubular wall, the stent is a suture-free stent.

In some embodiments the tubular wall includes a first end and a second end with the one or more leaflets disposed between the first end and the second end. In some embodiments the method also includes withdrawing the first mandrel from within the tubular wall *via* the first end, and withdrawing the second mandrel from within the tubular wall via the second end.

In some embodiments the method further includes seeding cells onto the polymeric fibers of the tubular wall.

### BRIEF DESCRIPTION OF THE DRAWINGS

Figure 1A schematically depicts a device and method for formation of polymeric fibers and collection of the polymeric fibers on a collection device to achieve different alignments or weaves of polymeric fibers on a collection device, for use in fabricating an engineered tubular structure in accordance with some embodiments of the invention.
Figure 1B schematically illustrates a relationship between mandrel rotation axis orientation during fiber collection and a resulting polymeric fiber orientation on a collection device for a mandrel rotation axis parallel to a deposition rotation axis.
Figure 1C schematically illustrates a relationship between mandrel rotation axis orientation during fiber collection and a resulting polymeric fiber orientation or weave on a collection device for a mandrel rotation axis at an angle relative to the deposition rotation axis.
Figure 2A is a perspective view of a first mandrel used for making an engineered valve having integral leaflets, in accordance with an embodiment.
Figure 2B is a perspective view of a second mandrel used, in combination with the first mandrel of Figure 2A, for making an engineered valve having integral leaflets.
Figure 3A is a perspective view of the first mandrel before collection of a first portion of polymeric fibers, illustrating rotation about a mandrel rotation axis in a method for making the engineered valve having integral leaflets, in accordance with an embodiment.
Figures 3B is a perspective view of the first mandrel after collection of the first portion of polymeric fibers on an outer surface of the first mandrel in the method for making the engineered valve having integral leaflets.
Figure 4A is a perspective view of the second mandrel positioned with respect to the first mandrel to form a composite mandrel illustrating rotation of the composite mandrel about a mandrel rotation axis in the method for making the engineered valve having integral leaflets.
Figure 4B is a perspective view of the combined mandrel after collection of a second portion of the polymeric fibers on an outer surface of the combined mandrel in the method for making an engineered valve having integral leaflets.
Figure 5 is a perspective view of a stent that can be embedded in a tubular wall of the resulting engineered valve having integral leaflets, in accordance with some embodiments.
Figure 6A is a perspective view of the composite mandrel with the stent positioned over the second portion of polymeric fibers in a method of making an engineered valve with integral leaflets and embedded stent.
Figure 6B is a perspective view of the composite mandrel having a third portion of polymeric fibers collected over the stent in the method of making an engineered valve with integral leaflets and embedded stent.
Figure 7 is a side cross-section view of the resulting engineered valve with integral leaflets and embedded stent of Figure 6B before separation of the engineered valve from the first and second mandrels.
Figure 8 is an image of collection of polymeric fibers on a combined mandrel for forming an example engineered valve with integral leaflets and an embedded stent using an automated system, in accordance with some embodiments.
Figure 9A is a perspective view image of an example engineered valve.
Figure 9B is an end view image of the example engineered valve with a first side of each leaflet in focus.
Figure 9C is an end view image of the example engineered valve with the embedded stent in focus.
Figure 9D is an end view image from the opposite end of the example engineered valve with a second side of each leaflet in focus.
Figure 10 schematically depicts a method of making a valve having integral leaflets, in accordance with another embodiment.
Figure 11 schematically depicts an automated system device for formation of polymeric fibers and collection of the polymeric fibers on a collection device, for use in fabricating an engineered valve structure in accordance with some embodiments of the invention.
Figure 12 is an image of a native leaflet of a human semilunar valve.
Figure 13 schematically depicts an exemplary mandrel design for forming integral leaflets, which is based upon the known measurements of human semilunar valve leaflet shown in Figure 10.

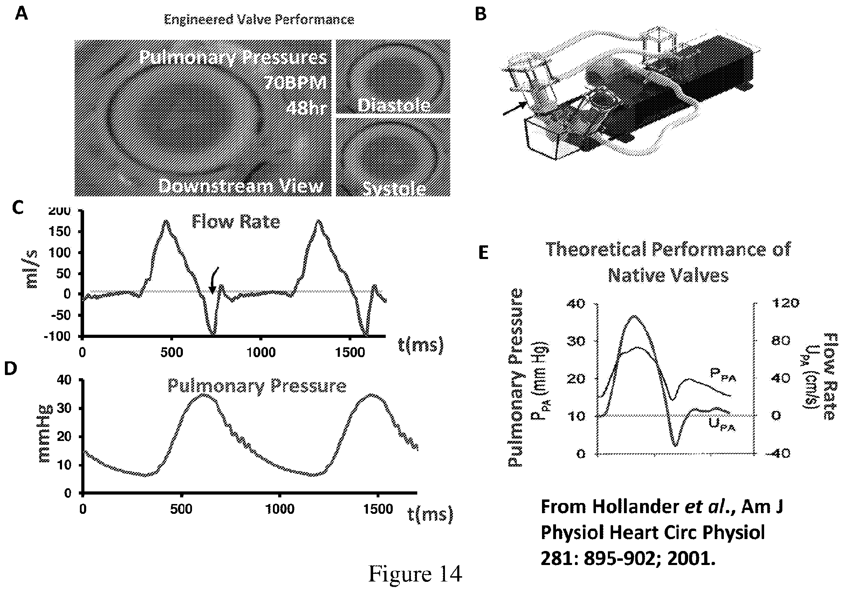

Figures 14A-14E depict the results of the *in vitro* testing of the engineered valves with integral leaflets: (A) is an image of an engineered valve mounted in a custom conduit mounting chamber of a Vivitro Inc. flow loop system during diastole and systole; (B) is a perspective view of a schematic of the flow loop system including the custom conduit mounting chamber (indicated by arrow) used for *in vitro* assessment of the valves of exemplary valves; (C and D) are a graphs of flow rate versus time and pulmonary pressure versus time, respectively, obtained under pulmonary conditions (appropriate heart pressures and flows using saline), demonstrating that the valve maintained the expected pressures during diastole with <20% closing volume at the beginning of diastole (arrow, C); and (E) is a graph depicting theoretical performance of native valves with respect to pulmonary pressure (P_{PA}, left axis) and flow rate (U_{PA}, right axis) versus time for comparison to the graphs in C and D.
Figures 15A-15C depict results from *in vivo* ovine testing after acute implantation of exemplary valves. Echo and Doppler measurements (A) show complete opening during systole (arrow, forward flow light gray-white) and closure during diastole (arrow, backward flow white). Explantation (at 15 hours) revealed no major clotting or loss of scaffold structure (B) and full cell penetration (C), light gray - cell nuclei; medium gray - red blood cells; and dark gray - gelatin/scaffold.

### DETAILED DESCRIPTION OF THE INVENTION

The present invention is based, at least in part, on the production and assembly of engineered tubular structures including oriented polymeric fibers that are suitable for use as, for example, engineered cardiac valves.

### I. Methods for Generating Engineered Valve Structures

Methods for generating engineered valve structures of the invention may include a step of configuring micron, submicron or nanometer dimension polymeric fibers in a desired shape.

Suitable devices and use of the devices for fabricating the micron, submicron or nanometer dimension polymeric fibers for use in the methods of the present invention are described in U.S. Patent Publication No. 2012/0135448, U.S. Patent Publication No. 2013/0312638, and U.S. Patent Publication No. 2014/0322515.

The micron, submicron or nanometer dimension polymeric fibers may have a diameter of about 15, 20, 25, 30, 35,40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 33, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nanometers, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 10, 20, 30, 40, or about 50 micrometers (µm). In some embodiments, the polymeric fibers have a diameter of about 0.5-3 µm. In some embodiments, the polymeric fibers have a diameter of about 0.7 to 1.3 µm. In some embodiments, an average diameter of the formed polymeric fibers is between about 0.75 µm and about 1.25 µm. In some embodiments the average diameter of the formed polymeric fibers is about 1µm. Ranges and values intermediate to the above recited ranges and values are also contemplated to be part of the invention.

Exemplary materials used to make fibers include synthetic polymers, such as polyethylene, polypropylene, poly(lactic acid), *etc.*

In some exemplary embodiments, the synthetic polymers may be specifically synthesized to possess domains along the backbone that may be activated for specific purposes including, but not limited to, specific binding, folding, unfolding, *etc.*

Exemplary materials may also include biogenic polymers, *e.g*., natural polymers, such as chitosan, alginate, gelatin, *etc.* Exemplary biogenic polymers may also include protein materials, such as collagen, fibronectin, laminin, *etc.* Exemplary materials may also include other suitable materials, *e.g*., metallic or ceramic materials.

Exemplary polymers for use in the methods of the invention may be biocompatible or nonbiocompatible, synthetic or natural, such as, for example, synthetic or natural polymers having shear induced unfolding, or combinations thereof. Exemplary polymers include, for example, poly(urethanes), poly(siloxanes) or silicones, poly(ethylene), poly(vinyl pyrrolidone), poly(2-hydroxy ethyl methacrylate), poly(N-vinyl pyrrolidone), poly(methyl methacrylate), poly(vinyl alcohol), poly(acrylic acid), polyacrylamide, poly(ethylene-co-vinyl acetate), poly(ethylene glycol), poly(methacrylic acid), polylactides (PLA), polyglycolides (PGA), poly(lactide-co-glycolides) (PLGA), polyanhydrides, polyphosphazenes, polygermanes, polyorthoesters, polyesters, polyamides, polyolefins, polycarbonates, polyaramides, polyimides, polycaprolactone (PCL), and copolymers and derivatives thereof, and combinations thereof.

Exemplary polymers for use in the methods of the invention may also be naturally occurring polymers *e.g.,* biogenic polymers, *e.g.,* proteins, polysaccharides, lipids, nucleic acids or combinations thereof.

Exemplary biogenic polymers, *e.g.,* polymers made in a living organism, *e.g.,* fibrous proteins, for use in the devices and methods of exemplary embodiments include, but are not limited to, silk (*e.g.,* fibroin, sericin, *etc.*)*,* keratins (*e.g.,* alpha-keratin which is the main protein component of hair, horns and nails, beta-keratin which is the main protein component of scales and claws, *etc.*)*,* elastins (*e.g.,* tropoelastin, *etc.*)*,* fibrillin (*e.g.,* fibrillin-1 which is the main component of microfibrils, fibrillin-2 which is a component in elastogenesis, fibrillin-3 which is found in the brain, fibrillin-4 which is a component in elastogenesis, *etc.*)*,* fibrinogen/fibrins/thrombin (*e.g.,* fibrinogen which is converted to fibrin by thrombin during wound healing), fibronectin, laminin, collagens (e.g., collagen I which is found in skin, tendons and bones, collagen II which is found in cartilage, collagen III which is found in connective tissue, collagen IV which is found in extracellular matrix protein, collagen V which is found in hair, *etc.*)*,* vimentin, neurofilaments (*e.g.,* light chain neurofilaments NF-L, medium chain neurofilaments NF-M, heavy chain neurofilaments NF-H, *etc.*)*,* amyloids (*e.g.,* alpha-amyloid, beta-amyloid, *etc.*)*,* actin, myosins (*e.g.,* myosin I-XVII, *etc.*)*,* titin which is the largest known protein (also known as connectin), gelatin, *etc.,* and combinations thereof.

Exemplary biogenic polymers, *e.g*., fibrous polysaccharides, for use in the present invention include, but are not limited to, chitin which is a major component of arthropod exoskeletons, hyaluronic acid which is found in extracellular space and cartilage (*e.g.,* D-glucuronic acid which is a component of hyaluronic acid, D-N-acetylglucosamine which is a component of hyaluronic acid, *etc.*)*, etc.*

Exemplary biogenic polymers, *e.g*., glycosaminoglycans (GAGs) (carbohydrate polymers found in the body), for use in the present invention include, but are not limited to, heparan sulfate founding extracelluar matrix, chondroitin sulfate which contributes to tendon and ligament strength, keratin sulfate which is found in extracellular matrix, *etc.*

In one embodiment, the polymers may be mixtures of two or more polymers and/or two or more copolymers. In one embodiment, the polymers may be a mixture of one or more polymers and or more copolymers. In another embodiment, the polymers for use in the devices and methods of the invention may be a mixture of one or more synthetic polymers and one or more naturally occurring polymers. For example, in one embodiment, the polymers are a mixture of collagen and polycarpolactone. In another embodiment, the polymers are a hybrid of poly-4-hydroxybutyrate (P4HB), polyglycolic acid (PGA), and gelatin/collagen. In yet another embodiment, the polymers are a mixture of polycaprolactone and gelatin, *e.g*., uncrosslinked gelatin.

In some embodiments, suitable devices for fabricating the micron, submicron or nanometer dimension polymeric fibers configured in a desired shape generally include a reservoir for holding a polymer, the reservoir including one or more orifices for ejecting the polymer during fiber formation, thereby forming a micron, submicron or nanometer dimension polymeric fiber and a collection device, *e.g.,* a mandrel, for accepting the formed micron, submicron or nanometer dimension polymeric fiber, wherein at least one of the reservoir and the collection device employs rotational motion during fiber formation, and the device is free of an electrical field, *e.g.,* a high voltage electrical field.

The device may include a rotary motion generator for imparting a rotational motion to the reservoir and, in some exemplary embodiments, to the collection device. In some embodiments, a flexible air foil is attached to a shaft of the motor above the reservoir to facilitate fiber collection and solvent evaporation.

Rotational speeds of the reservoir in exemplary embodiments may range from about 1,000 rpm-60,000 rpm, about 1,000 rpm-50,000 rpm, about 1,000 rpm to about 40,000 rpm, about 1,000 rpm-30,000 rpm, about 1,000 rpm to about 20,000 rpm, about 1,000 rpm-10,000 rpm, about 5,000 rpm-60,000 rpm, about 5,000 rpm-50,000 rpm, about 5,000 rpm to about 40,000 rpm, about 5,000 rpm-30,000 rpm, about 5,000 rpm-20,000 rpm, about 5,000 rpm to about 15,000 rpm, about 5,000 rpm-10,000 rpm, about 10,000 rpm-60,000 rpm, about 10,000 rpm-50,000 rpm, about 10,000 rpm to about 40,000 rpm, about 10,000 rpm-30,000 rpm, about 10,000 rpm-20,000 rpm, about 10,000 rpm to about 15,000 rpm, about 20,000 rpm-60,000 rpm, about 20,000 rpm-50,000 rpm, about 20,000 rpm to about 40,000 rpm, about 20,000 rpm-30,000 rpm, or about 50,000 rpm to about 400,000 rpm, *e.g*., about 1,000, 1,500, 2,000, 2,500, 3,000, 3,500, 4,000, 4,500, 5,000, 5,500, 6,000, 6,500, 7,000, 7,500, 8,000, 8,500, 9,000, 9,500,10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 20,500, 21,000, 21,500, 22,000, 22,500, 23,000, 23,500, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 55,000, 60,000, 65,000, 70,000, 75,000, 80,000, 85,000, 90,000, 95,000, 100,000, 105,000, 110,000, 115,000, 120,000, 125,000, 130,000, 135,000, 140,000, 145,000, 150,000 rpm, about 200,000 rpm, 250,000 rpm, 300,000 rpm, 350,000 rpm, or 400,000 rpm. Ranges and values intermediate to the above recited ranges and values are also contemplated to be part of the invention.

In certain embodiments, rotating speeds of about 50,000 rpm-400,000 rpm are intended to be encompassed by the methods of the invention. In one embodiment, devices employing rotational motion may be rotated at a speed greater than about 50, 000 rpm, greater than about 55,000 rpm, greater than about 60,000 rpm, greater than about 65,000 rpm, greater than about 70,000 rpm, greater than about 75,000 rpm, greater than about 80,000 rpm, greater than about 85,000 rpm, greater than about 90,000 rpm, greater than about 95,000 rpm, greater than about 100,000 rpm, greater than about 105,000 rpm, greater than about 110,000 rpm, greater than about 115,000 rpm, greater than about 120,000 rpm, greater than about 125,000 rpm, greater than about 130,000 rpm, greater than about 135,000 rpm, greater than about 140,000 rpm, greater than about 145,000 rpm, greater than about 150,000 rpm, greater than about 160,000 rpm, greater than about 165,000 rpm, greater than about 170,000 rpm, greater than about 175,000 rpm, greater than about 180,000 rpm, greater than about 185,000 rpm, greater than about 190,000 rpm, greater than about 195,000 rpm, greater than about 200,000 rpm, greater than about 250,000 rpm, greater than about 300,000 rpm, greater than about 350,000 rpm, or greater than about 400,000 rpm.

Exemplary devices employing rotational motion may be rotated for a time sufficient to form a desired polymeric fiber, such as, for example, about 1 minute to about 100 minutes, about 1 minute to about 60 minutes, about 10 minutes to about 60 minutes, about 30 minutes to about 60 minutes, about 1 minute to about 30 minutes, about 20 minutes to about 50 minutes, about 5 minutes to about 20 minutes, about 5 minutes to about 30 minutes, or about 15 minutes to about 30 minutes, about 5-100 minutes, about 10-100 minutes, about 20-100 minutes, about 30-100 minutes, or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 minutes, or more. Times and ranges intermediate to the above-recited values are also intended to be part of this invention.

In some embodiments, the reservoir may not be rotated, but may be pressurized to eject the polymer material from the reservoir through one or more orifices. For example, a mechanical pressurizer may be applied to one or more surfaces of the reservoir to decrease the volume of the reservoir, and thereby eject the material from the reservoir. In another exemplary embodiment, a fluid pressure may be introduced into the reservoir to pressurize the internal volume of the reservoir, and thereby eject the material from the reservoir.

An exemplary reservoir may have a volume ranging from about one nanoliter to about 1 milliliter, about one nanoliter to about 5 milliliters, about 1 nanoliter to about 100 milliliters, or about one microliter to about 100 milliliters, for holding the liquid material. Some exemplary volumes include, but are not limited to, about one nanoliter to about 1 milliliter, about one nanoliter to about 5 milliliters, about 1 nanoliter to about 100 milliliters, one microliter to about 100 microliters, about 1 milliliter to about 20 milliliters, about 20 milliliters to about 40 milliliters, about 40 milliliters to about 60 milliliters, about 60 milliliters to about 80 milliliters, about 80 milliliters to about 100 milliliters, but are not limited to these exemplary ranges. Exemplary volumes intermediate to the recited volumes are also part of the invention. In certain embodiment, the volume of the reservoir is less than about 5, less than about 4, less than about 3, less than about 2, or less than about 1 milliliter. In other embodiments, the physical size of an unfolded polymer and the desired number of polymers that will form a fiber dictate the smallest volume of the reservoir.

In one embodiment, a polymer is fed into a reservoir as a polymer solution, *i.e.,* a polymer dissolved in an appropriate solution. In this embodiment, the methods may further comprise dissolving the polymer in a solvent prior to feeding the polymer into the reservoir. In other embodiments, a polymer is fed into the reservoir as a polymer melt. In such embodiment, the reservoir is heated at a temperature suitable for melting the polymer, *e.g*., is heated at a temperature of about 100°C to about 300°C, 100-200°C, about 150-300°C, about 150-250°C, or about 150-200°C, or about 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or about 300°C.

The reservoir includes one or more orifices through which one or more jets of the material solution (*e.g*., polymer solution) are forced to exit the reservoir by the motion of the reservoir during fiber formation. One or more exemplary orifices may be provided on any suitable side or surface of the reservoir including, but not limited to, a bottom surface of the reservoir that faces the collection device, a side surface of the reservoir, a top surface of the reservoir that faces in the opposite direction to the collection device, *etc.* Exemplary orifices may have any suitable cross-sectional geometry including, but not limited to, circular, oval, square, rectangular, *etc.* In an exemplary embodiment, one or more nozzles may be provided associated with an exemplary orifice to provide control over one or more characteristics of the material solution exiting the reservoir through the orifice including, but not limited to, the flow rate, speed, direction, mass, shape and/or pressure of the material solution. The locations, cross-sectional geometries and arrangements of the orifices on the reservoir, and/or the locations, cross-sectional geometries and arrangements of the nozzles on the orifices, may be configured based on the desired characteristics of the resulting fibers and/or based on one or more other factors including, but not limited to, viscosity of the material solution, the rate of solvent evaporation during fiber formation, *etc.*

Exemplary orifice lengths that may be used in some exemplary embodiments range between about 0.001 m and about 0.05 m, *e.g.,* 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, or 0.05. In some embodiments, exemplary orifice lengths that may be used range between about 0.002 m and 0.01 m. Ranges and values intermediate to the above recited ranges and values are also contemplated to be part of the invention.

Exemplary orifice diameters that may be used in some exemplary embodiments range between about 0.1 µm and about 10 µm, about 50 µm to about 500 µm, about 200 µm to about 600 µm, about 200 µm to about 1,000 µm, about 500 µm to about 1,000 µm, about 200 µm to about 1,500 µm, about 200 µm to about 2,000 µm, about 500 µm to about 1,500 µm, or about 500 µm to about 2,000 µm, *e.g*., about 10, 20, 30, 40, 50, 100, 150, 200, 250, 300, 350, 400, 450, 500, 550, 600, 650, 700, 750, 800, 850, 900, 950, 1,000, 1,050, 1,100, 1,150, 1,200, 1,250, 1,300, 1,350, 1,400, 1,450, 1,500, 1,550, 1,600, 1,650, 1,700, 1,750, 1,800, 1,850, 1,900, 1,950, or about 2,000 µm. Ranges and values intermediate to the above recited ranges and values are also contemplated to be part of the invention.

In other embodiments, a suitable device for the formation of a micron, submicron or nanometer dimension polymeric fibers includes a reservoir for holding a polymer, the reservoir including one or more orifices for ejecting the polymer during fiber formation, thereby forming micron, submicron or nanometer dimension polymeric fibers, a collection device, *e.g.,* a mandrel, and an air vessel for circulating a vortex of air around the formed fibers to wind the fibers into one or more threads.

In yet other embodiments, a suitable device for the formation of a micron, submicron or nanometer dimension polymeric fiber includes a reservoir for holding a polymer, the reservoir including one or more orifices for ejecting the polymer during fiber formation, thereby forming a micron, submicron or nanometer dimension polymeric fiber, a collection device, *e.g.,* a mandrel, one or more mechanical members disposed or formed on or in the vicinity of the reservoir for increasing an air flow or an air turbulence experienced by the polymer ejected from the reservoir, and a collection device for accepting the formed micron, submicron or nanometer dimension polymeric fiber.

In one embodiment, a suitable device further comprises a component suitable for continuously feeding the polymer into the rotating reservoir (or a platform), such as a spout or syringe pump.

An exemplary method to fabricate the micron, submicron or nanometer dimension polymeric fibers configured in a desired shape (*e.g.,* a tubular shape, a valve shape) may include imparting rotational motion to a reservoir holding a polymer, the rotational motion causing the polymer to be ejected from one or more orifices in the reservoir and collecting the formed fibers on a mandrel having the desired shape, to form the micron, submicron or nanometer dimension polymeric fibers in the desired shape.

In one embodiment, the methods include feeding a polymer into a rotating reservoir of a device of the invention and providing motion at a speed and for a time sufficient to form a micron, submicron or nanometer dimension polymeric fiber, and collecting the formed fibers on a mandrel having the desired shape, to form the micron, submicron or nanometer dimension polymeric fibers in the desired shape.

In another embodiment, the methods include providing a polymer solution and imparting a sufficient amount of shear stress to the polymer solution for a time sufficient to form a micron, submicron or nanometer dimension polymeric fiber, and collecting the formed fibers on a mandrel having the desired shape, to form the micron, submicron or nanometer dimension polymeric fibers in the desired shape. In one embodiment, a sufficient amount of shear stress in about 3,000 Pascals.

In another embodiment, the methods include feeding a polymer solution into a rotating reservoir of a device of the invention and providing an amount of shear stress to the rotating polymer solution for a time sufficient to form a micron, submicron or nanometer dimension polymeric fiber, and collecting the formed fibers on a mandrel having the desired shape, to form the micron, submicron or nanometer dimension polymeric fibers in the desired shape.

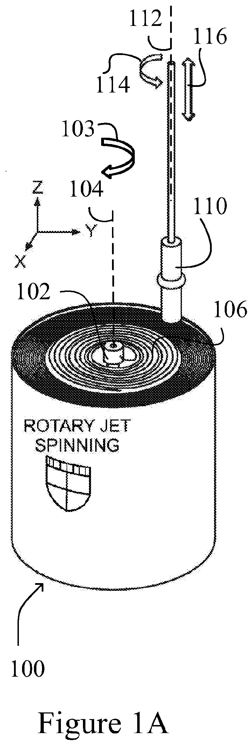

For example, Figure 1A schematically depicts a system 100 for forming micron, submicron or nanometer dimension polymeric fibers in accordance with some embodiments. System 100 includes a rotating reservoir 102 that rotates 103 about an axis, which is referred to as the deposition rotation axis 104. In Figures 1A-1C, the z-axis is defined to be parallel to deposition rotation axis 104 merely for convenience. One of ordinary skill in the art, in view of the present disclosure, would recognize that another convention may be chosen for describing an orientation of the deposition rotation axis, and that the deposition rotation axis need not be aligned with a vertical axis. The reservoir 102 has orifices through which polymer is ejected forming the polymeric fibers 106. As shown in Figure 1A, a collection device (*e.g*., mandrel 110) is be inserted into a path of the polymeric fibers 106. In some embodiments, the collection device (*e.g*., mandrel 110) is rotated 114 about an axis, which is referred to as the collection rotation axis 112. When the collection device is a mandrel, the collection rotation axis may be referred to as a mandrel rotation axis. When the collection device (*e.g*., mandrel 110) is in the path of the polymeric fibers 106 ejected from the rotating reservoir 102, the polymeric fibers 106 are wrapped around the collection device (*e.g.,* mandrel 110) via rotation (indicated by arrow 114) of the collection device about the collection rotation axis 112 as the collection device is translated (indicated by arrow 116) along the collection rotation axis 112.

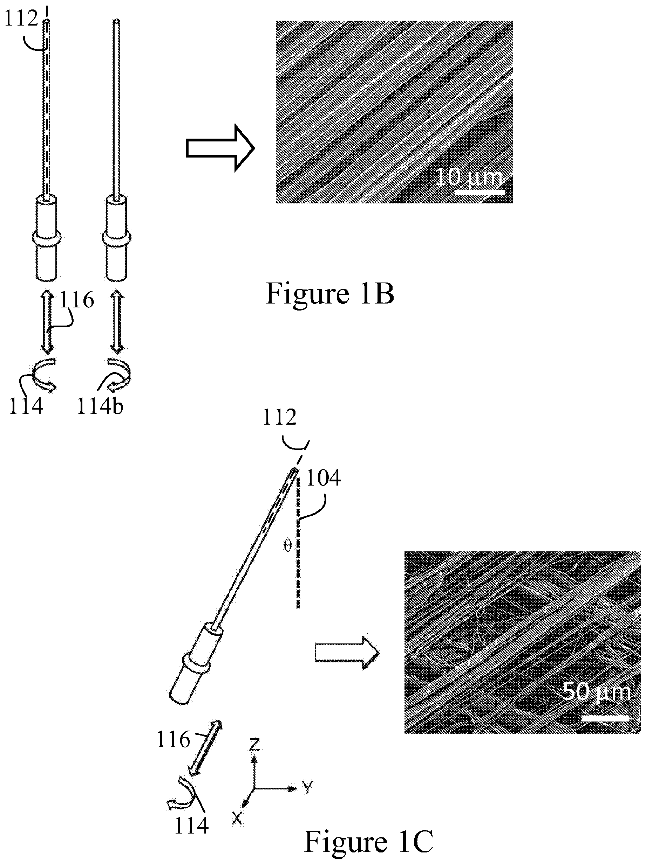

Control over the rate of translation 116 along the collection rotation axis 112 (or parallel to the deposition rotation axis 104) and orientation of the collection rotation axis 112 relative to the deposition rotation axis 104 provides control over an orientation of fibers deposited on the collection device (*e.g*., mandrel 110), as depicted in Figures 1B and 1C.

Figure 1B schematically illustrates collection of fibers with the collection rotation axis 112 parallel to the deposition rotation axis, which is the z-axis in this case, and with slow translation along the collection rotation axis (or parallel to the deposition rotation axis). The rotation 114 of the collection device (*e.g*., mandrel 110) may be opposite the rotation of the reservoir 105 (*e.g*., counter-clockwise and clockwise, respectively as shown) or the rotation of the collection device 114b may be the same as the rotation of the reservoir 105 (*e.g.,* both counter-clockwise). A scanning electron micrograph (SEM) of the polymeric fibers collected on the collection device show the orientation of polymeric fibers in the resulting structure. By slowly moving the collection device (*e.g*., mandrel 110) along the collection rotation axis 112 through a path of the polymeric fibers 106 during rotation of the collection device 114 or 114b, completely aligned coverage of the mandrel can be achieved. This may be described as complete anisotropy in fiber orientation.

Figure 1C schematically illustrates collection of fibers with the collection rotation axis 112 at an angle θ to the deposition rotation axis, which is the z-axis in this case. An SEM of the fibers collected on the collection device show the resulting structure with crossed polymeric fibers. By increasing speed of translation and/or rotating the mandrel at a nonzero angle θ with respect to the deposition rotation axis, cross or "x" shaped weaves can be produced. The mandrel may be moved manually or mechanically.

In other embodiments of the inventions, suitable devices for fabricating the micron, submicron or nanometer dimension polymeric fibers configured in a desired shape include a platform for supporting a stationary deposit of a polymer, a rotating structure disposed vertically above the platform and spaced from the platform along a vertical axis, and a collection device. The rotating structure includes a central core rotatable about a rotational axis, and one or more blades affixed to the rotating core. The rotating structure is configured and operable so that, upon rotation, the one or more blades contact a surface of the polymer to impart sufficient force in order to decouple a portion of the polymer from contact with the one or more blades of the rotating structure and to fling the portion of the polymer away from the contact with the one or more blades and from the deposit of the polymer, thereby forming a micron, submicron and/or nanometer dimension polymeric fiber.

Sufficient rotational speeds and times for operating the rotating structure to form a fiber may be dependent on the concentration of the material and the desired features of the formed fiber. Exemplary speeds of rotation of the rotating structure may range from about 100 rpm to about 500,000 rpm, although rotational speeds are not limited to this exemplary range. Certain exemplary devices employing rotational motion may be rotated at a speed of about 1,000 rpm-60,000 rpm, about 1,000 rpm-50,000 rpm, about 1,000 rpm to about 40,000 rpm, about 1,000 rpm-30,000 rpm, about 1,000 rpm to about 20,000 rpm, about 1,000 rpm-10,000 rpm, about 5,000 rpm-60,000 rpm, about 5,000 rpm-50,000 rpm, about 5,000 rpm to about 40,000 rpm, about 5,000 rpm-30,000 rpm, about 5,000 rpm-20,000 rpm, about 5,000 rpm to about 15,000 rpm, about 5,000 rpm-10,000 rpm, about 10,000 rpm-60,000 rpm, about 10,000 rpm-50,000 rpm, about 10,000 rpm to about 40,000 rpm, about 10,000 rpm-30,000 rpm, about 10,000 rpm-20,000 rpm, about 10,000 rpm to about 15,000 rpm, about 20,000 rpm-60,000 rpm, about 20,000 rpm-50,000 rpm, about 20,000 rpm to about 40,000 rpm, about 20,000 rpm-30,000 rpm, or about 50,000 rpm to about 400,000 rpm, *e.g.,* about 1,000, 1,500, 2,000, 2,500, 3,000, 3,500, 4,000, 4,500, 5,000, 5,500, 6,000, 6,500, 7,000, 7,500, 8,000, 8,500, 9,000, 9,500,10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 20,500, 21,000, 21,500, 22,000, 22,500, 23,000, 23,500, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 55,000, 60,000, 65,000, 70,000, 75,000, 80,000, 85,000, 90,000, 95,000, 100,000, 105,000, 110,000, 115,000, 120,000, 125,000, 130,000, 135,000, 140,000, 145,000, 150,000 rpm, about 200,000 rpm, 250,000 rpm, 300,000 rpm, 350,000 rpm, or 400,000 rpm. Ranges and values intermediate to the above recited ranges and values are also contemplated to be part of the invention. For example, rotating speeds of about 10,000 rpm-15,000 rpm, or 8,000 rpm-12,000 rpm are intended to be encompassed by the methods of the invention.

In one embodiment, devices employing rotational motion may be rotated at a speed greater than about 1, 000 rpm, greater than about 1,500 rpm, greater than about 2,000 rpm, greater than about 2,500 rpm, greater than about 3,000 rpm, greater than about 3,050 rpm, greater than about 3,100 rpm, greater than about 3,150 rpm, greater than about 3,200 rpm, greater than about 3,250 rpm, greater than about 3,300 rpm, greater than about 3,350 rpm, greater than about 3,400 rpm, greater than about 3,450 rpm, greater than about 3,500 rpm, greater than about 3,550 rpm, greater than about 3,600 rpm, greater than about 3,650 rpm, greater than about 3,700 rpm, greater than about 3,750 rpm, greater than about 3,800 rpm, greater than about 3,850 rpm, greater than about 3,900 rpm, greater than about 3,950 rpm, or greater than about 4,000 rpm. Speeds intermediate to the above recited speeds are also contemplated to be part of the invention.

An exemplary rotating structure may be rotated to impact the liquid material for a time sufficient to form a desired fiber, such as, for example, about 1 minute to about 100 minutes, about 1 minute to about 60 minutes, about 10 minutes to about 60 minutes, about 30 minutes to about 60 minutes, about 1 minute to about 30 minutes, about 20 minutes to about 50 minutes, about 5 minutes to about 20 minutes, about 5 minutes to about 30 minutes, or about 15 minutes to about 30 minutes, about 5-100 minutes, about 10-100 minutes, about 20-100 minutes, about 30-100 minutes, or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 minutes, or more. Times and ranges intermediate to the above-recited values are also intended to be part of this invention.

The one or more portions or components of the rotating structure may penetrate into the surface of the liquid material to a desired depth. Exemplary depths of penetration may range from about one nanometer to about one centimeter, but are not limited to this range. Some exemplary penetration depths include, but are not limited to, about one millimeter to about twenty milliliters, about twenty milliliters to about forty milliliters, about forty milliliters to about sixty milliliters, about sixty milliliters to about eighty milliliters, about eighty milliliters to about one hundred milliliters, about one centimeter, and the like. Exemplary penetration depths intermediate to the above-recited exemplary values are also intended to be part of this invention.

The rotating structure may be configured in any suitable manner so that, upon rotation, the rotating structure contacts a surface of the liquid material on platform to impart sufficient force or energy to create a meniscus at the location where the rotating structure contacts the surface. The force or energy imparted by the rotating structure overcomes the surface tension and decouples a portion of the liquid material at the meniscus and flings the portion away from the contact with the rotating structure and from the platform, thereby forming a micron, submicron and/or nanometer dimension fiber. The fiber may be collected on the collection device. In an exemplary embodiment, the direction in which the liquid material is flung may be substantially the same as the tangential direction of motion of the component of the rotating structure that contacts the liquid material. In an exemplary embodiment, the rotating structure may impart a force to the liquid material in a substantially parallel direction to the top surface of the liquid material.

In one embodiment, the rotating structure may have a central core rotatable in a clockwise and/or counter-clockwise manner about a central axis of rotation R. In an exemplary embodiment, the rotational axis R may be offset at substantially 90 degrees from the vertical axis V. The core may have a substantially cylindrical shape with a substantially circular cross-section having a center aligned along the axis of rotation R. The rotating structure may also include one or more protrusions, *e.g*., in the form of blades, brushes, bristles, *etc.,* affixed to the outer surface of the rotating core so that part of the protrusions penetrate into the surface of the liquid material. Exemplary rotating structures may include any suitable number of protrusions affixed to the core including, but not limited to, one protrusion to 500 protrusions. Some exemplary numbers of protrusions include, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, about 50 to about 100, about 100 to about 200, about 200 to about 300, about 300 to about 400, about 400 to about 500, and the like. Exemplary numbers of protrusions intermediate to the recited exemplary numbers are also part of the invention. The protrusions may be configured on the core in any suitable arrangement including, but not limited to, a regular multi-row or multi-column arrangement, an array pattern, a circular arrangement, a random arrangement, and the like.

Each protrusion may have any suitable shape including, but not limited to, a substantially rectangular shaped protrusion, a saw shaped protrusion wherein the base of the protrusion at the core is wider than the tip farthest from the core, a cylindrical shaped protrusion, and the like. At high rotational speeds and/or in instances where broken protrusions would compromise the purity of the fibers, the saw shape may provide enhanced structural integrity to the protrusions and may prevent break-off of the protrusions during rotation.

In some exemplary embodiments, one or more aspects of the protrusions on the rotating structure may be varied to control the adherence of the liquid material to the protrusions when the liquid material comes into contact with the protrusions, thereby facilitating fiber formation. Exemplary aspects that may be control or configured include, but are not limited to, the surface chemistry of the protrusions, the surface topography of the protrusions (*e.g.,* a rougher texture), a geometry of the protrusions (*e.g.,* a cross-sectional shape of the protrusions), and the like. In addition, configuring these aspects of the protrusions may allow controlling the geometry of the fibers that are formed, fiber width, surface features on the fibers, and the like.

Exemplary protrusions may be formed of any suitable material including, but not limited to, titanium, stainless steel (*e.g.,* 300 and 400 alloys), aluminum (*e.g.,* 6061, 7075), polystyrene, polypropylene, (*e.g*., UHMW, HDPE, LDPE), ABS, acetal (copolymer and homopolymer), nylon, polycarbonate, polyether ether ketone, polymethyl methacrylate, polysulfone, polytetrafluoroethylene, polyvinylchloride, and the like.

Any suitable size or geometrically shaped reservoir or collector may be used for fabricating polymeric fibers. For example, the reservoir may be tubular, conical, semilunar, bicuspid, round, rectangular, or oval. The collector (and/or the mandrel) may be round, oval, rectangular, or a half-heart shape. The collector may also be shaped in the form of any living organ, such as a heart, kidney, liver lobe(s), bladder, uterus, intestine, skeletal muscle, or lung shape, or portion thereof. The collector may further be shaped as any hollow cavity, organ or tissue, such as a circular muscle structure, *e.g*., a valve, sphincter or iris.

In some embodiments in which a tubular structure or engineered valve is fabricated, the collection device may be a mandrel configured in a desired shape and positioned in the path of the polymer ejected from the one or more orifices or in the path of the fibers flung from the rotating structure.

In exemplary embodiments, the collection device, *e.g*., mandrel, may be disposed at a distance of about 2 inches (about 5 cm) to about 12 inches (about 30 cm) from the reservoir from which the polymer is ejected, but is not limited to this exemplary range. Certain exemplary distances may include, but are not limited to 2, 3, 4, 5, 6, 7, 8, 9, 10, 11, 12 inches or 5, 7.6, 10.2, 12.7, 15.2, 17.8, 20.3, 22.9, 25.4, 27.9, 30 cm, and all intermediate numbers, and the like.

This distance is carefully configured to avoid formation of fibrous beads (which may occur if the collection device is too close to the reservoir) and to achieve sufficient fibrous mass (which may not occur if the collection device is too far from the reservoir).

An exemplary collection device, *e.g*., mandrel, may be coupled to one or more motion generators for imparting rotational and/or linear motion to the collection device. An exemplary collection device may be rotated about at speeds ranging from about 1,000 rpm to about 80,000 rpm, but is not limited to this exemplary range. Rotational speeds of the collection device in exemplary embodiments may range from about 1,000 rpm-50,000 rpm, about 1,000 rpm to about 40,000 rpm, about 1,000 rpm to about 20,000 rpm, about 5,000 rpm-20,000 rpm, about 5,000 rpm to about 15,000 rpm, or about 50,000 rpm to about 400,000 rpm, *e.g*., about 1,000, 1,500, 2,000, 2,500, 3,000, 3,500, 4,000, 4,500, 5,000, 5,500, 6,000, 6,500, 7,000, 7,500, 8,000, 8,500, 9,000, 9,500,10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, 20,000, 20,500, 21,000, 21,500, 22,000, 22,500, 23,000, 23,500, or about 24,000, 50,000, 55,000, 60,000, 65,000, 70,000, 75,000, 80,000, 85,000, 90,000, 95,000, 100,000, 105,000, 110,000, 115,000, 120,000, 125,000, 130,000, 135,000, 140,000, 145,000, 150,000 rpm, about 200,000 rpm, 250,000 rpm, 300,000 rpm, 350,000 rpm, or 400,000 rpm. Ranges and values intermediate to the above recited ranges and values are also contemplated to be part of the invention.

In other embodiments, an exemplary collection device, *e.g.,* a mandrel, may be coupled to one or more motion generators for imparting rotational motion to the collection device and rotated about at speeds ranging from about 1,000 rpm to about 6,000 rpm, *e.g.,* about 1,100 rpm, about 1,200 rpm, about 1,300 rpm, about 1,400 rpm, about 1,500 rpm, about 1,600 rpm, about 1,700 rpm, about 1,800 rpm, about 1,900 rpm, about 2,000 rpm, about 2,100 rpm, about 2,200 rpm, about 2,300 rpm, about 2,400 rpm, about 2,500 rpm, about 2,600 rpm, about 2,700 rpm, about 2,800 rpm, about 2,900 rpm, about 3,000 rpm, about 3,100 rpm, about 3,200 rpm, about 3,300 rpm, about 3,400 rpm, about 3,500 rpm, about 3,600 rpm, about 3,700 rpm, about 3,800 rpm, about 3,900 rpm, about 4,000 rpm, about 4,100 rpm, about 4,200 rpm, about 4,300 rpm, about 4,400 rpm, about 4,500 rpm, about 4,600 rpm, about 4,700 rpm, about 4,800 rpm, about 4,900 rpm, about 5,000 rpm, about 5,100 rpm, about 5,200 rpm, about 5,300 rpm, about 5,400 rpm, about 5,500 rpm, about 5,600 rpm, about 5,700 rpm, about 5,800 rpm, about 5,900 rpm, or about 6,000 rpm. Increased rotational speeds may enable greater fiber alignment.

An exemplary collection device, *e.g*., mandrel, may be linearly translated relative to a rotational axis of a rotating structure or rotating reservoir of a fiber formation system (*e.g*., translated back and forth along an axis parallel to the rotation axis of the rotating structure or rotating reservoir of the fiber formation system or translated back and forth along an axis at an angle to the rotational axis of the rotating structure or rotating reservoir) at linear speeds ranging from about 1 mm/s to about 300 mm/s, *e.g*., about 1, 2, 3, 4, 5, 6, 7, 8, 9, 10, 20, 25, 30, 35, 40 4, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, or about 300 mm/s. In some embodiments, the mandrel is linearly translated at speeds ranging from about 10 mm/s to about 200 mm/s. Ranges and speeds intermediate to the recited ranges and speeds are also contemplated by the present invention. In some embodiments, the rotating structure or rotating reservoir of the fiber formation system may also, or alternatively, be translated relative to the collection mandrel during collection of the fibers. The translation of the collection mandrel relative to the rotating structure or rotating reservoir may bring the collection mandrel in and out of the plane through which the flung or ejected fibers travel (*i.e.,* the fiber plane) to ensure complete fiber coverage.

An exemplary engineered valve (and tubular structure) may be formed using any suitable number of overlapping layers of polymeric fibers. Exemplary numbers of layers of polymeric fibers of about 5 to about 1,000,000 may be disposed on the mandrel at a time to create a polymeric valve structure. In some embodiments, exemplary numbers of layers of polymeric fibers fall in a range of 5 to 5,000,000.

An exemplary valve (and tubular structure) structure may have any desired length and thickness. Exemplary thicknesses may range from about 50 microns to about 2 mm, but are not limited to this exemplary range. In some embodiments, exemplary thicknesses may range from about 1 micron to about 5 mm. The thickness is carefully configured to be sufficient to provide mechanical and tensile strength to the valve (and tubular structure) and/or so that the valve (and tubular structure) allows diffusion of cells and nutrition to the inner layers of the polymeric fibers. In order to fabricate the valve (and tubular structure) structure in a desired shape, the mandrel is positioned in the path of the fibers ejected from the reservoir or in the path of the fibers flung from the rotating structure and rotated, angled and/or vertically maneuvered such that the fibers are accepted on the mandrel at a desired thickness and pattern. In one embodiment, the maneuvering of the mandrel is automated.

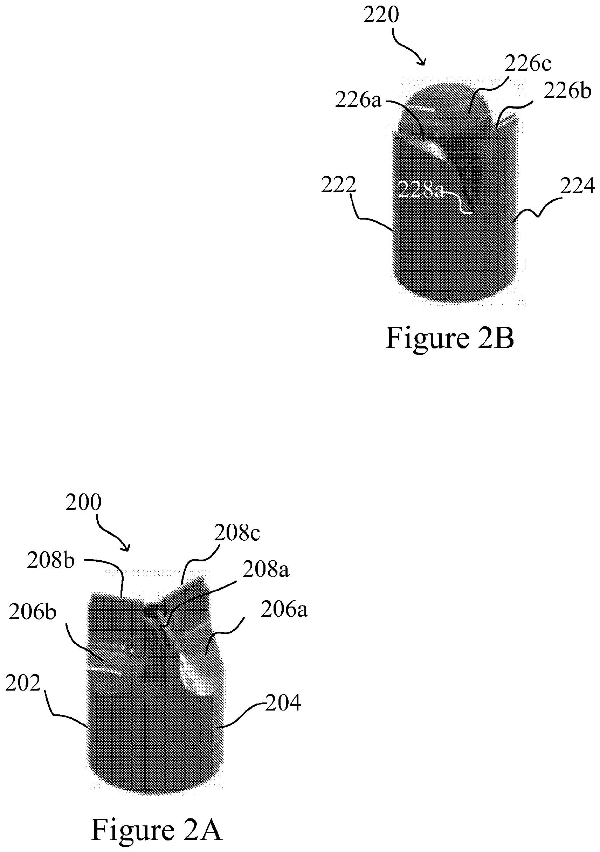

In one embodiment, the mandrel is in a tubular shape. In another embodiment, the mandrel is in a conical shape. In other embodiments, the mandrel is configured in the shape of a tricuspid or bicuspid valve. In some embodiments, a collection device in the form of a multi-part mandrel may be employed. Portions of the multi-part mandrel may together form a combined mandrel. In some embodiments, polymeric fibers are collected on one or portions of the multi-part mandrel in one or more steps, and collected on the combined mandrel in one or more other steps. For example, a multi-part mandrel may be used to fabricate an engineered valve having a tubular wall and one or more leaflets integral with and extending from an inner surface of the tubular wall. An exemplary method of making such an engineered valve having integral leaflets using a multi-part mandrel is explained below with reference to Figures 2A-7.

Figures 2A and 2B depict the structure of an exemplary multi-part mandrel for forming an engineered valve having a tubular wall with integral leaflets. The multi-part mandrel includes a first mandrel 200 and a second mandrel 220 that together form a combined mandrel 230.

The first mandrel 200 has an outer surface 202 with a first tubular forming portion 204 and one or more leaflet forming portions 206a, 206b. The first tubular forming portion 204 has a shape corresponding to an inner surface of a first portion of the resulting tubular wall. As depicted, the first tubular forming portion 204 has a cylindrical shape; however, in other embodiments the shape may be non-cylindrical (*e.g*., a cross-sectional shape of the first tubular forming portion may be oval, elliptical, irregular, and/or changing along a length of the portion). Each of the one or more leaflet portions 206a, 206b of the outer surface of the first mandrel has a shape corresponding to a first surface of a corresponding one of the one or more leaflets of the resulting valve.

The second mandrel 220 has an outer surface 222 including a second tubular forming portion 224 and one or more leaflet forming portions 226a, 226b, 226c. The second tubular forming 224 portion has a shape corresponding to an inner surface of a second portion of the resulting tubular wall. The one or more leaflet forming portions 226a, 226b, 226c may each have a shape corresponding to a second surface of a corresponding one of the one or more leaflets of the resulting valve.

As depicted in Figures 2A and 2B, the first mandrel has three leaflet forming portions and the second mandrel has corresponding three leaflet forming portions and the resulting valve is a tricuspid valve; however, in other embodiments, the first mandrel may include more or fewer than three leaflet portions (*e.g*., 1, 2, 4, 5, 6, 7, 8, 9, 10, 11, or 12 leaflet portions).

As depicted in Figures 2A and 2B, each leaflet forming portion 206a, 206b of the first mandrel has a concave shape, and each leaflet forming portion of the second mandrel has a convex shape 228a, 228b, 228c. However, as would be recognized by one of ordinary skill in the art in view of the present disclosure, the leaflet forming portions of the first mandrel may have a shape other than concave, (*e.g.,* a planar shape, a partially planar and partially concave shape). The leaflet forming portions of the second mandrel may have a shape other than convex (*e.g.,* a planar shape, a partially planar and partially convex shape, *etc.*)*.*

In some embodiments, the first mandrel 200 includes one or more first engagement portions 208a, 208b, 208c and the second mandrel includes one or more second engagement portions 228a that each engage a corresponding one of the first engagement portions. Such engagement portions may aid fixing a position of the first mandrel relative 200 to the second mandrel 220, and vice versa, during collection of polymeric fibers on the composite mandrel. For example, as depicted, the one or more first engagement portions 208a, 208b, 208c each include a protrusion of the first mandrel and the one or more second engagement portions 228a each include a recess in the second mandrel configured to receive a corresponding protrusion. In other embodiments, the first mandrel may include engagement portions in the form of one or more recesses and the second mandrel may include engagement portions in the form of one or more corresponding protrusions.

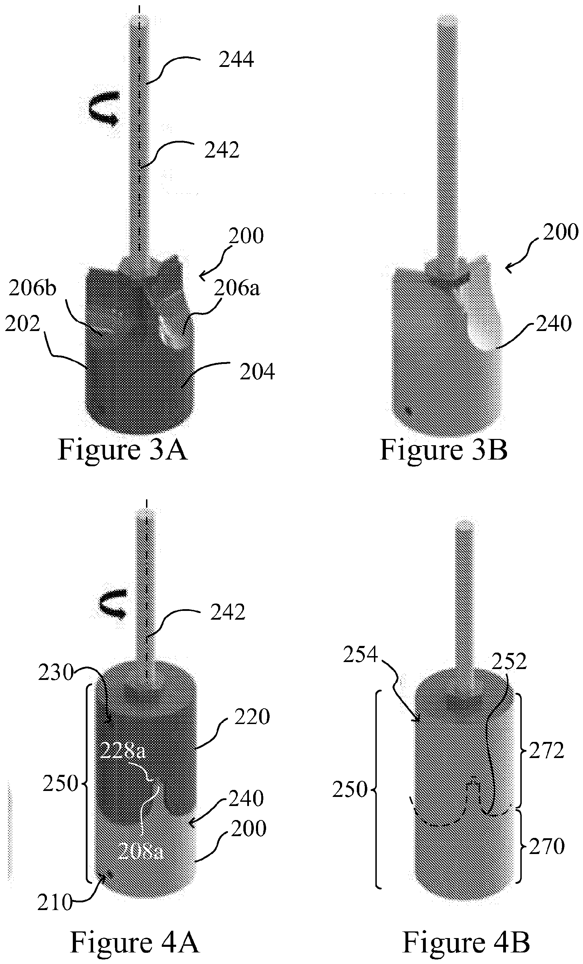

A method of forming an exemplary valve having a tubular wall and integral leaflets are schematically depicted in Figures 3A-7. Initially, a first portion of polymeric fibers including micron, submicron and/or nanometer dimension fibers are collected on the outer surface 202 of the first mandrel as shown in Figures 3A and 3B. As used herein, collection of polymeric fibers on a collection device may also be referred to deposition of the polymeric fibers on the collection device. Figure 3A depicts the first mandrel 200 prior to collection of the first portion of the polymeric fibers and Figure 3B depicts the first mandrel 200 after collection of the first portion of the polymeric fibers 240 on an outer surface 202 of the first mandrel.

The polymeric fibers are formed by ejecting or flinging a polymer from reservoir (*e.g.,* as described above with respect to Figures 1A-1C). In some embodiments, the first portion of the polymeric fibers 240 is collected on the first mandrel 200 by positioning the first mandrel 200 in a path of the ejected or flung polymeric particles. In an exemplary method, the first mandrel 200 in the path of the ejected or flung polymeric particles is rotated about a mandrel rotation axis 242 using a linear member 244 connected to a motor (not shown) during collection. During collection of the first portion of the polymeric fibers, an orientation of the mandrel rotation axis 242 with respect to a deposition rotation axis (*see* deposition rotation axis 104 of Figure 1A) and/or a rate of translation along the mandrel rotation axis (or parallel to the deposition rotation axis 104) may be selected to achieve a desired orientation or structure of the collected polymeric articles on the first mandrel. An orientation of the mandrel rotation axis relative to the deposition rotation axis falls in a range of 0 to 180 degrees. In some embodiments, the orientation falls in a range of 5 to 85, 10 to 80, 15 to 75, 20 to 70, 25 to 65, 30 to 60, 35 to 55, 40 to 50, or 42 to 48 degrees.

The first portion of the polymeric fibers 240 collected on the first mandrel at least partially forms one or more leaflets of the resulting valve. Specifically, the first portion of the polymeric fibers 240 forms at least a first surface of each leaflet, the first surface being the surface adjacent to the corresponding leaflet forming portion 206a, 206b of the outer surface of the first mandrel 200.

The first portion of the polymeric fibers 240 collected on the first mandrel also at least partially forms a first portion of the resulting tubular wall. Specifically, the first portion of the polymeric fibers 240 forms, at least, an inner surface of a first portion of the tubular wall that connects with the at least partially formed leaflets. As shown in Figure 3B, most or all of the outer surface 202 of the first mandrel is covered by the first portion of the polymeric fibers. However, in other embodiments, the first portion may only cover the leaflet forming portions 206a, 206b, and at least a part of the first tubular forming portion 204 adjacent to the leaflet forming portions 206a, 206b.

The deposition of the first portion 240 of the polymeric fibers on the first mandrel 200 forms at least an interior surface of a portion of the resulting tubular wall and forms at least a portion of the resulting one or more leaflets. As such, the outer shape 202 of the first mandrel defines the shape of the interior surface of the first portion of the resulting tubular wall and the shape of the first surface of each leaflet.

Some of the polymeric fibers in the first portion deposited on the first mandrel extend from a leaflet forming portion 206a, 206b to the tubular forming portion 204 of the mandrel surface. These fibers are part of a resulting leaflet as well as part of the resulting tubular wall. Thus, in the resulting valve, the resulting leaflets will be integrated with the first portion of the resulting tubular wall adjacent to the leaflets.

After the first portion of the polymeric fibers is deposited on the first mandrel 200, the second mandrel 220 is positioned relative to the first mandrel to form a combined mandrel 250, as shown in Figure 4A. In the combined mandrel 250, first engagement portions 208a of the first mandrel may engage corresponding second engagement portions 228a of the second mandrel to secure a position of the first mandrel 200 relative to the second mandrel 220. In the combined mandrel 250, the first portion 240 of the polymeric fibers is disposed between the first mandrel 200 and the second mandrel 220 and on the tubular forming portion 204 of the outer surface of the first mandrel. The combined mandrel 250 forms a crevasse between the first mandrel 200 and the second mandrel 220. The first portion of the polymeric fibers 240 is disposed within the crevasse and extends, at least, to the boundary between the crevasse and the first tubular forming portion 204 of the first mandrel.

A second portion of the polymeric fibers including micron, submicron and/or nanometer dimension fibers is collected on an outer surface of the combined mandrel 250. Figure 4A depicts the combined mandrel 250 prior to deposition of the second portion of the polymeric fibers. Figure 4B depicts the combined mandrel first mandrel 250 after collection of the second first portion 260 of the polymeric fibers on an outer surface of the combined mandrel. The boundary between the first mandrel 200 and the second mandrel 220, which is covered by the second portion of the polymeric fibers, is indicated with the dotted line 252. The polymeric fibers are formed by ejecting or flinging a polymer from a reservoir as described with respect to Figures 1A-1C.

In some embodiments, the second portion 254 of the polymeric fibers is collected on the combined mandrel 250 by positioning the combined mandrel 250 in a path of the ejected or flung polymeric particles. In an exemplary method, the first mandrel 200 in the path of the ejected or flung polymeric particles is rotated about a mandrel rotation axis 242 using the linear member 244 connected to a motor (not shown) during collection. During collection of the second portion of the polymeric fibers, the orientation of the mandrel rotation axis 242 with respect to a deposition rotation axis (see deposition rotation axis 104 of Figure 1A) and/or a rate of translation along the mandrel rotation axis (or parallel to the deposition rotation axis) may be selected to achieve a desired orientation or structure of the collected polymeric articles on the combined mandrel.

In some embodiments, the polymer used for the polymeric fibers of the second portion is the same as the polymer used to form the polymeric fibers of the first portion. In some embodiments, a first polymer is used to make the polymeric fibers of the first portion and a different second polymer is used for the polymeric fibers of the second portion. In some embodiments, different polymer materials may be deposited during collection of a portion of the polymeric fibers. For example, the deposition of the first portion to form leaflet may include depositing first a thin layer of elastin and collagen followed by a layer of glycosaminoglycans and ending with a thicker layer of collagen. In some embodiments, the polymer solution is constantly fed into the reservoir and extruded, which permits continuous adjustments of the polymer solution materials. In some embodiments, a layer of antibacterial fibers may be deposited as an outer layer on the combined mandrel to help maintain sterility. In some embodiments, a diameter of the fibers produced fall in a range of 5-300 nm.

In some embodiments, the orientation of the mandrel rotation axis relative to the deposition rotation axis is the same during collection of the first portion on the first mandrel and collection of the second portion on the second mandrel. In some embodiments, the orientation of the mandrel rotation axis relative to the deposition rotation axis is different for collection of the first portion on the first mandrel and collection of the second portion on the second mandrel.

In some embodiments, the mandrel rotation rate may be fall in a range of 100-10000 rpm. In some embodiments, the mandrel rotation rate is the same during collection of the first portion on the first mandrel and collection of the second portion on the second mandrel. In some embodiments, the mandrel rotation rate is different for collection of the first portion on the first mandrel and collection of the second portion on the second mandrel.

As depicted in Figure 4B, the collected second portion 254 of the polymeric fibers overlies the first portion to further form a first wall portion 270 of the tubular wall overlying the first mandrel and forms a second wall portion 272 of the tubular wall overlying the second mandrel. In some embodiments, the first wall portion 270 and the second wall portion 272 together form the tubular wall of the resulting valve.

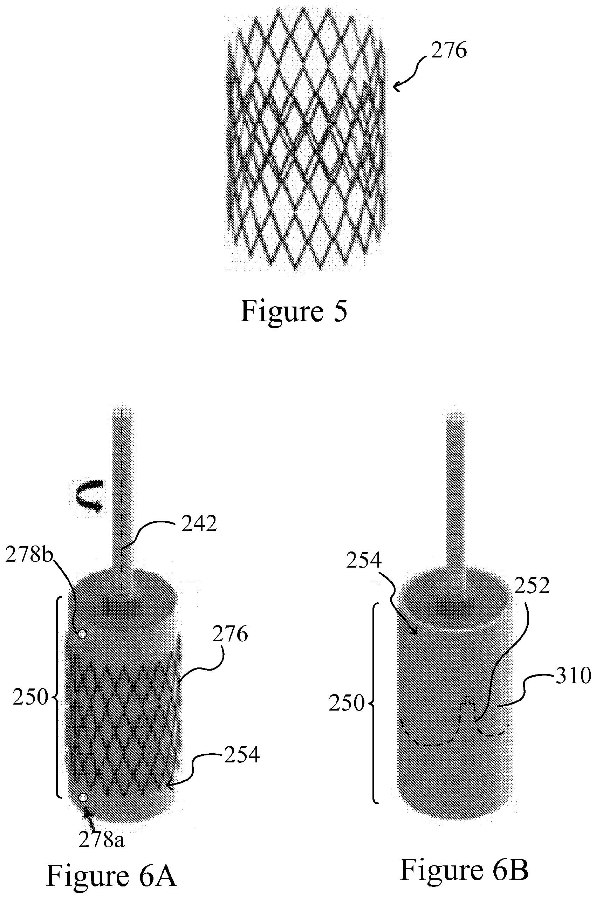

In some embodiments, an element (*e.g.,* a stent) is embedded in the tubular wall of the resulting valve. For example, Figure 5 depicts a stent 276 that can be embedded in a resulting tubular wall. Figures 6A and 6B depict embedding the stent in the tubular wall of the resulting valve.

The stent 276 is positioned on the composite mandrel over at least some of the second portion of polymeric fibers that were previously collected on the combined mandrel (*e.g*., slid onto the composite mandrel) as shown in Figure 6A. In some embodiments, one or more of the first mandrel and the second mandrel may include one or more features for securing an element, such as the stent, to the composite mandrel. For example, as shown Figure 4A, the first mandrel 200 includes a first screw hole 210 and the second mandrel 220 includes a second screw hole 230. In Figure 6A, set screws 278a, 278b are used with the first and second screw holes 210, 230 to secure the stent 276 to the combined mandrel 250.

A third portion 280 of the polymeric fibers are collected on an outside surface of the stent 254 to form the tubular wall 310 with the stent 276 embedded therein. Figure 6A depicts the composite mandrel 250 and the stent 276 prior to deposition of the third portion of the polymeric fibers, and Figure 6B depicts the composite mandrel 250 after collection of the third portion 280 of the polymeric fibers on the composite mandrel 250 over the second portion and over the stent.

The polymeric fibers are formed by ejecting or flinging a polymer from reservoir as described with respect to Figures 1A-1C. In some embodiments, the third portion of the polymeric fibers is collected on the composite mandrel 250 and stent 276 by positioning the composite mandrel 250 and stent 276 a path of the ejected or flung polymeric particles. In an exemplary method, the composite mandrel 250 and stent 276 in the path of the ejected or flung polymeric fibers are rotated about a mandrel rotation axis 242. During collection of the third portion of the polymeric fibers, an orientation of the mandrel rotation axis with respect to a deposition rotation axis and/or a translation rate of the compound mandrel along the mandrel rotation axis (or parallel to the deposition rotation axis) may be selected to achieve a desired orientation or structure of the collected polymeric articles on the combined mandrel.

In some embodiments, the polymer used for the polymeric fibers of the third portion is the same as the polymer used to form the polymeric fibers of the first portion and or the polymer used to form the polymeric fibers of the second portion. In some embodiments, the polymer used to make the polymeric fibers of the third portion is different from the polymer used to make the first portion and/or the polymer used to make the second portion.

In some embodiments, the orientation of the mandrel rotation axis relative to the deposition rotation axis is the same during collection of the collection of the third portion, as during collection of the second portion. In some embodiments, the orientation of the mandrel rotation axis relative to the deposition rotation axis is different for collection of the third portion than for collection of the second portion.

In some embodiments, the mandrel rotation rate is the same during collection of the third portion and during collection of the second portion. In some embodiments, the mandrel rotation rate is different for collection of the third portion and for collection of the second portion.

One of skill in the art, in view of the present disclosure, would appreciate that any appropriate element (*e.g*., medical device) could be embedded within the tubular wall, and the element need not be a stent. Other elements that may be embedded in the tubular wall include, but are not limited to: pressure transducers, time-released therapeutic agents (*e.g.,* therapeutic agents in capsules or pouches for treatment of atherosclerosis); flow sensors; actuators (*e.g.,* to expand or contract the conduit); optical tracers (*e.g.* in capsules or pouches, which could be activated or released for flow imaging).

Multiple elements could be embedded in the same layer (*e.g*., multiple elements could be positioned over the first portion and under the second portion of polymeric fibers). One of skill in the art, in view of the present disclosure, would recognize that elements could be embedded in different layers. A first stent could be positioned on the combined mandrel after collection of the first portion, a second stent could be positioned on the combined mandrel after collection of some of the second portion, with the rest of the second portion being deposited over the second stent. This would result in the first stent being embedded between the first portion and the second portion, and the second stent being embedded in a layer partially through the second portion.

Although, Figures 6A and 6B depict embedding one stent in the tubular wall, it should be understood that any number of stents (and/or any number of other appropriate devices) could be embedded within the tubular wall while remaining within the scope of the present disclosure. For example, in some embodiments, multiple stents may be embedded in the tubular wall.

During collection of fibers for the second portion and or the third portion the layers of fibers need not be collected equally across the surfaces of the mandrels 200, 210. For example, the fibers could be spun such that the tubular wall of the resulting valve is thicker in some portions than others. For example, the motors controlling the positioning of the first mandrel 200 or the composite mandrel 250 could cause some portions of the mandrels to be oriented for fiber collection for longer periods of time compared to others. Additionally or alternatively, when some portions of the mandrel are oriented for fiber collection, the polymer could be ejected from the reservoir at a faster rate than when other portions are so oriented.

After deposition of the second portion, in the case of no embedded device, or after deposition of the third portion, in the case of an embedded device, the first mandrel 100 and second mandrel 200 can be withdrawn through opposite ends of the formed tubular wall 310 to release the resulting valve from the combined mandrel 250. In some embodiments, the mandrels 200, 220 are pre-coated with a substance (*e.g*., a pre-coating substance comprising 1% gelatin) prior to the fiber collection to facilitate removal of the mandrels 200, 220 from the resulting valve. After completing formation of the valve 310 on the combined mandrel by deposition of the third portion of the polymeric fibers, the valve 310 and combined mandrel 250 can be placed in a fluid (e.g., water) to dissolve at least part of the pre-coating substance, thus loosening the mandrels 200, 210 for easy removal. Other substances that could be used for pre-coating include, but are not limited to: sugars (*e.g*., sucrose, glucose), fibronectin, collagen, any extracellular matrix protein, oils, salts (*e.g*., NaCl). In some embodiments, a pre-coating is not needed to release the valve from the mandrels.

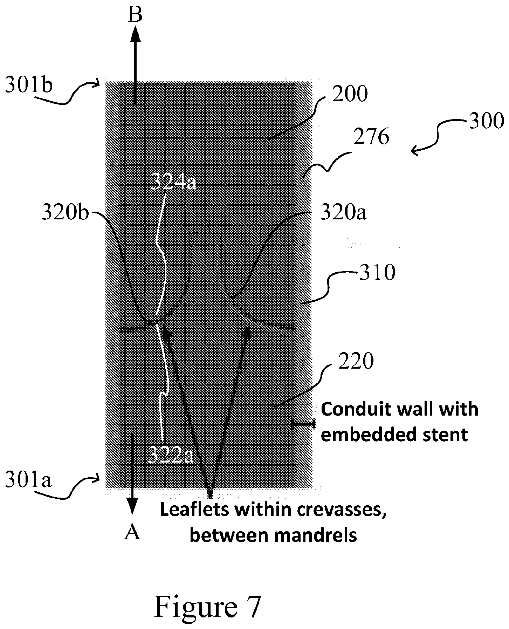

Figure 7 is a cross-sectional view of the resulting valve 300 before the first mandrel 200 and the second mandrel 220 are withdrawn. The tubular wall 310 is formed around the first tubular forming portion 204 of the first mandrel and the second tubular forming portion 224 of the second mandrel, which is an outer surface of the combined mandrel 250. The tubular wall may be described as a conduit wall in some embodiments. The leaflets 320a, 320 extend from an inner surface of the tubular wall 310. As shown in Figure 7, the leaflets 320a, 320b are disposed in a crevasse formed between the first mandrel 220 and the second mandrel 220. As shown, the first mandrel 200 controls a shape of a first surface 322a of each leaflet and the second mandrel 220 controls a shape of a second surface 324a of each leaflet.

The polymeric fibers define a shape of the tubular wall and of the leaflets. The leaflets 320a, 320b are integral with an inner surface of the tubular wall 310. At least some of the polymeric fibers form part of a leaflet 320a, 320b and part of the tubular wall 310. In other words, at least some of the polymer fibers of the tubular wall 310 interpenetrate with the polymer fibers of the leaflets 320a, 320b. More specifically, at least some of the polymer fibers of the leaflets 320a, 320b interpenetrate with at least some of the polymer fibers of the inner surface of the tubular wall 310. At the boundary between the inner surface of the tubular wall 310 and the leaflets 320a, 320b where the leaflets 320a, 320b, meet the inner surface, the polymer fibers of the leaflets 320a, 320b interpenetrate with the polymer fibers of the inner surface of the tubular wall 310.

Although the polymer fibers are collected in multiple different portions, the resulting tubular wall and leaflet structure is an integral structure, not a layered structure in which various layers are joined together with adhesive, sutures, welds, etc., *e.g.,* a suture-free valve. Further, the leaflets are integral with the tubular wall, not joined to a portion of the tubular wall with adhesive, sutures, welds, etc., *e.g.,* a suture-free valve. Thus, because the tubular wall is an integral structure and the leaflets 320a, 320b are integral with the inner surface of the tubular wall 310, the leaflets are also integral with the outer surface of the tubular wall 310.

As shown in Figure 7, the stent 276 is embedded in the tubular wall 310 in accordance with some embodiments.

As noted above, the mandrels 200, 220 can be separated from the resulting valve 300 without damaging the leaflet structure by withdrawing the first mandrel 200 from a first end 301a of the resulting valve in the direction indicated by arrow A and withdrawing the second mandrel 220 from a second end 301b of the resulting valve in the direction indicated by arrow B.

In the embodiment described above, the leaflet forming portions are recesses in the outer surface of the first mandrel and the second mandrel. In other embodiments, the first mandrel and/or second mandrel may include projections in addition to, or instead of, recesses. For example, the first mandrel and/or second mandrel may include protrusions that form sinuses of valves. As another example, the first mandrel and/or second mandrel may include protrusions to form contoured blood vessels.

### II. Example Valve with Embedded Stent and Integral Leaflets

An example valve with an embedded stent and integral leaflets was made using the method described above with respect to Figures 2A to 6B. Specifically, polymeric fibers were formed by ejection of 6% by weight solution of polycaprolactone (PCL) in hexafluoroisopropanol (HFIP) from a reservoir being rotated at 30,000 rotations per minute (rpm) through orifices of about 340 µm in diameter. The polymeric fibers were collected on the first mandrel and on the combined mandrel as described above. During collection of polymeric fibers, the mandrel was rotated at 3,000 rpm about a mandrel rotation axis at an angle of about 45 degrees with respect to the deposition rotation axis of the reservoir. During deposition the mandrel was translated parallel to the deposition rotation axis (*i.e.,* in a vertical direction).

The first portion of the polymeric fibers was collected on the first mandrel over about 5 minutes with the resulting collected first portion forming a layer about 0.5 mm thick on the first mandrel. The second portion of the polymeric fibers was collected on the combined mandrel over about 10 minutes with the resulting collected second portion forming a layer about 1.0 mm thick on the combined mandrel. After collection of the second portion, a nitinol stent was positioned over the second portion and secured to the combined mandrel. The third portion of the polymeric fibers was collected on the combined mandrel and stent over about 5 minutes with the resulting collected third portion forming a layer about 0.5 mm thick. A pre-coating substance comprising 1% gelatin was deposited/formed on the first and second mandrel before collection of the fibers to facilitate release of the resulting valve from the mandrels. Each fiber had a diameter of about 1 micron (+/- 250 nm).

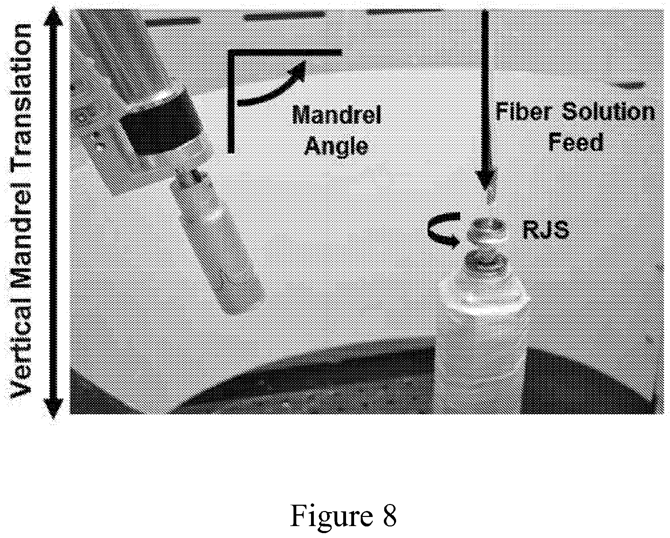

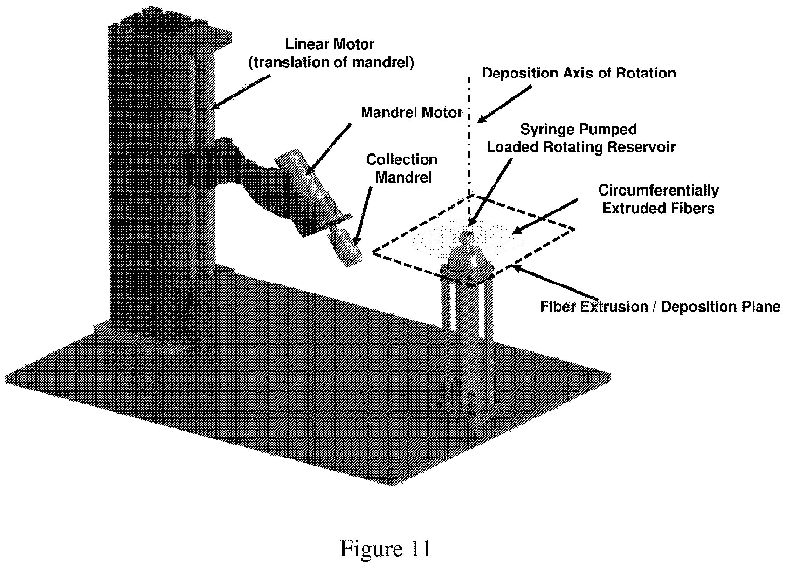

The collection of the fibers was performed by an automated system, shown in Figure 8, which controlled the rotation rate of the reservoir (*i.e.,* the rotary jet spinner (RJS)), the rotation rate of the mandrel, and vertical translation of the rotating mandrel (*see also* the schematic of Figure 11 which provides another view of the rotating reservoir and the motors used to rotate and translate the collection mandrel). In the system, a pneumatic rotary motor drove the rotation of the reservoir, which extruded the polymer solution through a small orifice. The extrusion produced a plane of fibers into which the rotation mandrel was translated in to and out of during the collection process in a customizable translation sequence. A direct current (DC) motor drove the rotation of the mandrel and was mounted onto a uniaxial high precision linear drive that translated the rotating mandrel along an axis parallel to the axis of rotation of the reservoir, in this case vertically. In other embodiments, one or more additional linear drives could be employed to translate the rotating mandrel along one or more axis perpendicular to the axis of rotation of the reservoir (*e.g*., movement toward and away from the deposition rotation axis). In other embodiments, a multi-axial drive or a robotic arm could be employed for greater flexibility in translation and/or changing an angular alignment of the mandrel.

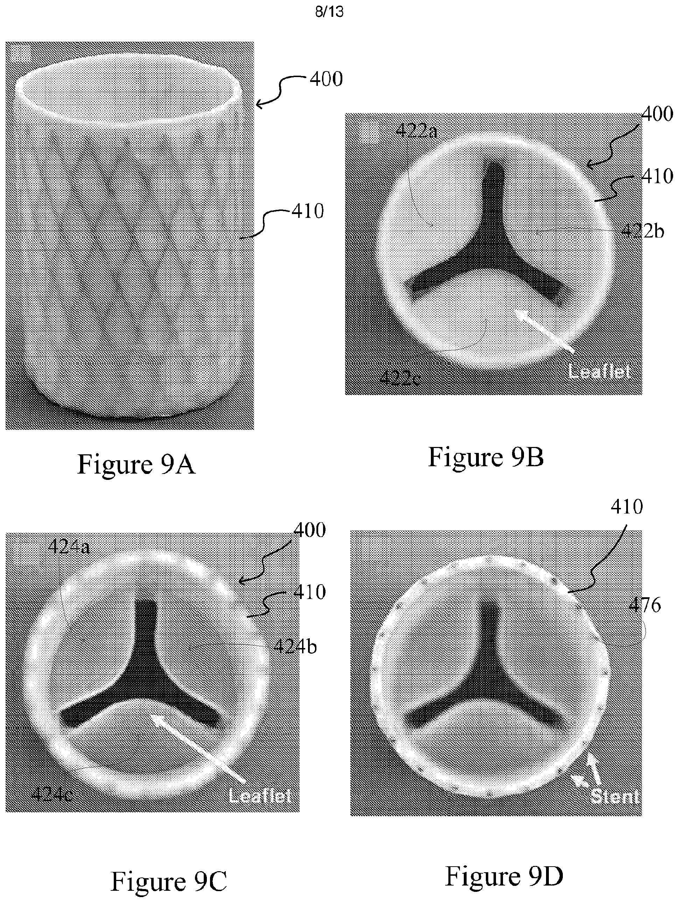

The resulting valve 400, is depicted in Figures 9A-9C. The first mandrel used to form the valve had an axial length of 20 mm. The second mandrel used to form the valve had an axial length of 25 mm. Both the first mandrel and the second mandrel had a diameter of 27 mm in the tubular forming portion. The stent embedded in the valve has a length of 38 mm, an inner diameter of 29.5 mm and an outer diameter of 30.0 mm. In other embodiments, the length of the first mandrel may be significantly longer than, or shorter than, the length of the second mandrel to position the leaflets closer to one end of the resulting valve.

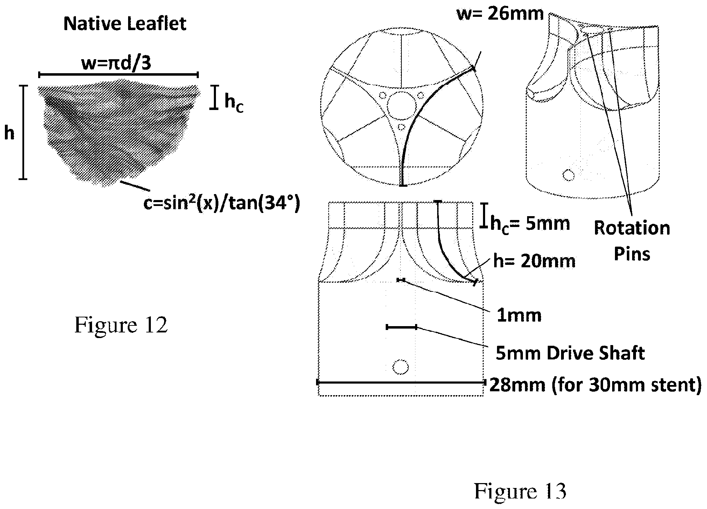

The length of the combined mandrel and the length of the resulting valve may be selected based on the planned use of the valve. In some embodiments, the combined mandrel may have a length in the range of 0.5 cm to 50 cm. The outer diameter of the combined mandrel and the inner diameter of the resulting valve may be selected based on the planned use of the valve. In some embodiments, the combined mandrel may have an outer diameter in the range of 5 mm to 100 mm. In some embodiments, the combined mandrel may have an outer diameter greater than 100 mm. Figure 13, which is discussed below, includes schematic diagrams of another mandrel used to form leaflets.

Figures 9A-9D are images of the example engineered valve 400 having integral leaflets and an embedded stent. Figure 9A shows a tubular wall 410 of the example engineered valve 400. Figure 9B is a first end view image of the example engineered valve showing a first surface 422a, 422b, 422c of each leaflet. Figure 9C is a second end view image of the example engineered valve focusing on a second surface 424a, 424b, 424c of each leaflet. Figure 9D is a second end view image of the example engineered valve focusing on an embedded stent 476. If the example engineered valve is used as an aortic valve, Figure 9B could be described as showing the ventricular side of the leaflets, and Figures 9C and 9D could be described as shown the aortic side of the leaflets. As shown in Figures 9A-9D, because the leaflets are formed integral with a portion of the tubular wall instead of formed and later joined to the tubular wall or to the stent embedded in the tubular wall, the example engineered valve 400 does not include any form of attachment means (*e.g*., sutures, glue) for attaching the leaflets to the conduit wall 410, nor does it contain any points of attachment (*e.g.,* such as those created via melt pressing).

The resulting valve with embedded stent may be described as a suture-free valve, because the leaflets are integral with the tubular wall, as opposed to attached to the tubular wall and stent using sutures, glue, or melt pressing. Such an integral valve may exhibit greater structural integrity and reliability due to reduction of stress at the connection between the leaflets and tubular structure as compared with a valve that experiences relatively high stress at points of attachment between the leaflets and the tubular structure. Because the leaflets are not attached to the tubular wall via suturing, melting, *etc.* the material of the valve is more uniform promoting more ingrowth of normal tissue, as opposed to the formation of scar tissue due to material mismatch.

Further, the tubular wall itself is an integral whole in which the stent is embedded. In other words, the three portions of collected fibers form an integral tubular wall instead of three separate layers of tubular wall that would need to be attached to each other and/or to the stent using sutures, glue, or melt pressing. Such an integral wall would likely exhibit greater structural integrity than layers connected to each other or to a stent only at discrete points. Further, the material of the tubular wall is more uniform than material that would results from multiple layers attached to each other only at discrete points, which promotes ingrowth of normal tissue.

The structure of the resulting valve may be configured to form a polymeric fiber scaffold for cellular ingrowth (described below).

One of ordinary skill in the art in view of the present disclosure would recognize that different sizes of mandrels and different shapes, sizes or numbers of leaflets could be employed to create different sizes of valves and/or different types of valves. Further, the leaflet may have different sizes or shapes. For example, the first and second mandrels could be selected to produce a valve with a specified inner diameter and specified leaflet shapes. Further, the mandrels may be designed with variances in their shapes, curvatures, and/or diameters for specific applications, or to accommodate the anatomy or needs of a particular patient or type of patient (*e.g.,* a child).

### III. Example Valve with Integral Leaflets

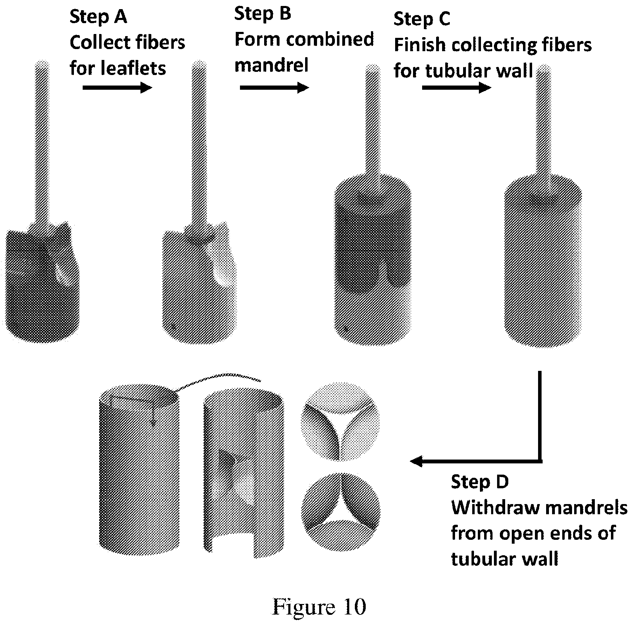

An exemplary valve with integral leaflets was made using a method similar to that described above with respect to Figures 2A to 6B. Figure 10 schematically depicts steps in forming the exemplary valve with integral leaflets and schematic views of the resulting valve. Specifically, polymeric fibers were formed by ejection of a polymer solution comprising 4% by weight total solute, which was 60% polyester, polycaprolactone (PCL), and 40% uncrosslinked gelatin, in hexafluoroisopropanol (HFIP) solvent. The polymer solution was ejected from a reservoir being rotated at about 30,000 rotations per minute (rpm) through orifices of about 340 µm in diameter and about 5 mm in length. A first portion of the polymeric fibers were collected on the first mandrel to form a portion of the inner surface of the tubular wall and to form the leaflets (Step A). The second mandrel was joined to the first mandrel, which included the collected first portion of the polymeric fibers, to form the combined mandrel (Step B). A second portion of the polymeric fibers were collected on the combined mandrel to complete formation of the tubular wall (Step C). The mandrels were then withdrawn from the open ends of the tubular wall yielding the valve with integral leaflets (Step D).

During collection of the first portion of polymeric fibers, approximately 30 ml of polymer solution was continuously pumped into the rotating reservoir, 10 ml at a time, pumped at a rate of approximately 5 ml/minute, and the mandrel was rotated at about 3,000 rpm about a mandrel rotation axis at an angle of about 45 degrees with respect to the deposition rotation axis of the reservoir. However, in other embodiments, the mandrel rotation axis may be at an angle of less than 45 degrees or greater than 45 degrees with respect to the rotation axis of the reservoir. Each fiber had a diameter of about 1 micron (+/-250 nm).

During deposition, the mandrel was linearly translated perpendicular to and through the fiber extrusion /deposition plane (e.g., in a vertical direction) while rotating about an axis at an angle of about 45 degrees with respect to the fiber extrusion/deposition plane. The fiber extrusion/deposition plane is perpendicular to the deposition rotation axis. Thus the mandrel was translated parallel to the deposition rotation axis (*i.e.,* in a vertical direction) while rotating about an axis at an angle of about 45 degrees with respect to the deposition rotation axis during deposition. The first portion of the polymeric fibers formed a layer about 0.5 mm thick on the first mandrel.

After a sufficient time to allow any volatile fumes to dissipate (*e.g*., about 5 minutes), excess fibers spun over the top of the mandrel were removed (*e.g*., with a scalpel or razor blade) and it was confirmed that the leaflets were not connected to each other and that each leaflet moved independently of the other leaflets. If needed, the spun fibers were pressed into the leaflet forming portions of the first mandrel to ensure that the spun fibers conformed to the shape of the leaflet forming portions of the mandrel before the second mandrel was joined to the first mandrel to form the combined mandrel.

The second portion of the polymeric fibers was collected on the combined mandrel using the same polyester/gelatin solution used for the first portion of polymeric fibers. During collection of the second portion of polymeric fibers, approximately 40 ml of polymer solution was continuously pumped into the rotating reservoir, rotating at about 30,000 rpm, 10 ml at a time, pumped at a rate of approximately 5 ml/minute, and the mandrel was rotated at about 3,000 rpm about a mandrel rotation axis at an angle of about 45 degrees with respect to the deposition rotation axis of the reservoir. During deposition of the second portion of polymeric fibers, the mandrel was linearly translated perpendicular to the fiber extrusion/deposition plane(*e.g*., in a vertical direction) while being rotated about an axis at about a 45 degree angle to the deposition plane. The second portion of the polymeric fibers formed a layer about 0.5 mm thick on the combined mandrel.

One of ordinary skill in the art would recognize that, as described above, after collection of a second portion of polymeric fibers, a stent may be positioned over the second portion of polymeric fibers and secured to the combined mandrel. A third portion of the polymeric fibers may then be collected on the combined mandrel and stent over, for example, about 5 minutes with the resulting collected third portion forming a layer about 0.5 mm thick.

The collection of the fibers was performed by an automated system, shown in Figure 11, which controlled the rotation rate of the reservoir (*i.e.,* the rotary jet spinner (RJS)), the rotation rate of the mandrel, and linear translation of the rotating mandrel along an axis parallel to the rotation axis of the rotating reservoir. In the system, a pneumatic rotary motor drove the rotation of the reservoir, which extruded the polymer solution through a small orifice. The extrusion produced a plane of fibers into which the rotation mandrel was translated into and out of during the collection process in a customizable translation sequence. A DC motor drove the rotation of the mandrel and was mounted onto a uniaxial high precision linear drive that translated the rotating mandrel along an axis parallel to the rotation axis of the rotating reservoir, which in this case is translation vertically. In other embodiments, one or more additional linear drives could be employed to translate the rotating mandrel along one or more axes perpendicular to the rotation axis of the rotating reservoirs (*e.g*., movement toward and away from the deposition rotation axis). In other embodiments, a multi-axial drive or a robotic arm could be employed for greater flexibility in translation and/or changing an angular alignment of the mandrel.

The first mandrel used to form the valve had an axial length of 20 mm. The second mandrel used to form the valve had an axial length of 25 mm. Both the first mandrel and the second mandrel had a diameter of 28 mm in the tubular forming portion. In other embodiments, the length of the first mandrel may be significantly longer than, or shorter than, the length of the second mandrel to position the leaflets closer to one end of the resulting valve. In some embodiments, the length of either or both of the mandrels may be between 5 mm and 30mm.

The length of the combined mandrel and the length of the resulting valve may be selected based on the planned use of the valve. In some embodiments, the combined mandrel may have a length in the range of 0.5 cm to 50 cm. The outer diameter of the combined mandrel and the inner diameter of the resulting valve may be selected based on the planned use of the valve. In some embodiments, the combined mandrel may have an outer diameter in the range of 5 mm to 100 mm. In some embodiments, the combined mandrel may have an outer diameter greater than 100 mm.

Because the leaflets are formed integral with a portion of the tubular wall instead of formed and later joined to the tubular wall, the example engineered valve does not include any form of attachment means (*e.g*., sutures, glue) for attaching the leaflets to the conduit wall, nor does it contain any points of attachment (*e.g*., such as those created via melt pressing).

The resulting valve may be described as a suture-free valve, because the leaflets are integral with the tubular wall, as opposed to attached to the tubular wall and stent using sutures, glue, or melt pressing. Such an integral valve may exhibit greater structural integrity and reliability due to reduction of stress at the connection between the leaflets and tubular structure as compared with a valve that experiences relatively high stress at points of attachment between the leaflets and the tubular structure. Because the leaflets are not attached to the tubular wall via suturing, melting, *etc.* the material of the valve is more uniform promoting more ingrowth of normal tissue, as opposed to the formation of scar tissue due to material mismatch.

In some embodiments, the resulting valve may be used with a stent (*e.g.,* the valve conduit may be inserted with the lumen of a stent). In some embodiments, the resulting valve may be secured to an outer stent through attachment means (*e.g*., via anchor sutures). Even if the valve is attached to a stent using anchor sutures, the valve itself would be a suture free valve because no attachment means are required to secure the leaflets to the tubular wall of the valve. Further, far fewer attachment points would be required to secure the suture-free valve to an external stent, as compared with attachment points required to secure leaflets to an outer conduit or stent in a conventional valve by, *e.g.,* the use of continuous sutures. As such, the attachments to secure the suture-free valve to a stent (*e.g*. anchor sutures, not continuous sutures) would cause less interference with tissue ingrowth than the interference cause by attachments to secure leaflets to a valve conduit or stent in a conventional valve. In some embodiments, friction may be sufficient such that attachments (*e.g*., anchor sutures) are not required to secure the tubular wall of the valve to the stent. In other embodiments, the stent is embedded in the tubular wall, as described above.

The structure of the resulting valve may be configured to form a polymeric fiber scaffold for cellular ingrowth (described below).

One of ordinary skill in the art in view of the present disclosure would recognize that different sizes of mandrels and different shapes, sizes or numbers of leaflets could be employed to create different sizes of valves and/or different types of valves. Further, the leaflet may have different sizes or shapes. For example, the first and second mandrels could be selected to produce a valve with a specified inner diameter and specified leaflet shapes. Further, the mandrels may be designed with variances in their shapes, curvatures, and/or diameters for specific applications, or to accommodate the anatomy or needs of a particular patient or type of patient (*e.g.,* a child).

In some embodiments, the dimensions of, for example, the first mandrel which forms the leaflets of the valve structure may, be based on the known measurements of the adult human semilunar valve. Figure 12 is an image of a native leaflet, on which the design of a mandrel may be based. The image is reproduced from "Straightening of Curved Pattern of Collagen Fibers Under Load Controls Aortic Valve Shape," by Peter E. Hammer, Christina A. Pacak, Robert E. Howe, and Pedro J. del Nido in Journal of Biomechanics, volume 47, issue 22, pages 341-346 (2014). Example dimensions for the leaflet shown in Figure 12 appear in the table below based on information from in Hammer *et al.,* 2014 and from "Dimensions and Geometric Relationships of the Human Aortic Valve as a Function of

Pressure," by Milton Swanson and Richard E Clark, Circulation Research, volume 35, issue 6, pages 871-882 (1974).

| **Dimensions for Adult Human Semilunar Valve** | |
|---|---|
| Height (h) | 19.2 mm |
| Width (w) w=πd/3 | 28.78 mm |
| Coaptation Height (h_{C}) *[Hammer et al.,* 2012] | 5 mm |
| Curvature below commissure (c) *[Swanson and Clark,* 1974] | c=sin²(x)/tan(34°) where x is the distance along the curvature or contour of the leaflet |

Figure 13 schematically depicts the dimensions of a first mandrel with dimensions based on the know measurements of the human semilunar valve describe above, whose native leaflet is shown in Figure 12.

Engineered valves, *i.e.,* semilunar valves with integral leaflets fabricated as described herein were evaluated *in vitro* under conditions mimicking those found in the adult human heart using a commercially available flow loop system (see, *e.g.,* Figure 12B). The valves were formed with dimensions corresponding to human semilunar valves according to the method described above with respect to Figure 10. By mounting the valve within a custom built mounting chamber in the flow loop system and using saline and right heart pressure and flow rates it was demonstrated that the valves maintained the expected pressures during diastole with <20% closing volume at the beginning of diastole (arrow, Figure 12C). In addition, the flow and pressure performance of the valves (Figure 12D) were comparable to the theoretical performance of native valves (Figure 12E). The chart for the theoretical performance of native valves is reproduced from Hollander et al., "Negative wave reflections in pulmonary arteries," Am J Physiol Heart Circ Physiol 281: 895-902; 2001.

The ability of cells to migrate into and grow within the valves was also assessed *in vitro.* Engineered valves, *i.e.,* semilunar valves with integral leaflets fabricated as described herein, were cultured with porcine valve interstitial cells or porcine valve endothelial cells, both of which were obtained by primary harvest, or human mesenchymal stem cells which were purchased from a commercial vendor. The porcine valve endothelial cells and the engineered valves were cultured in endothelial cell growth media, specifically EBM2 from Lonza Group Ltd of Basel, Switzerland. The human mesenchymal stem cells and the engineered valves were cultured in mesenchymal stem cell growth media, specifically MSCGM from Lonza. The porcine valve interstitial cells and the engineered valves were cultured in M119 + 10% fetal bovine serum. The results of these experiments demonstrate that the engineered valves with integral leaflets fabricated as described herein permit cell attachment and migration, and support cell and tissue growth.

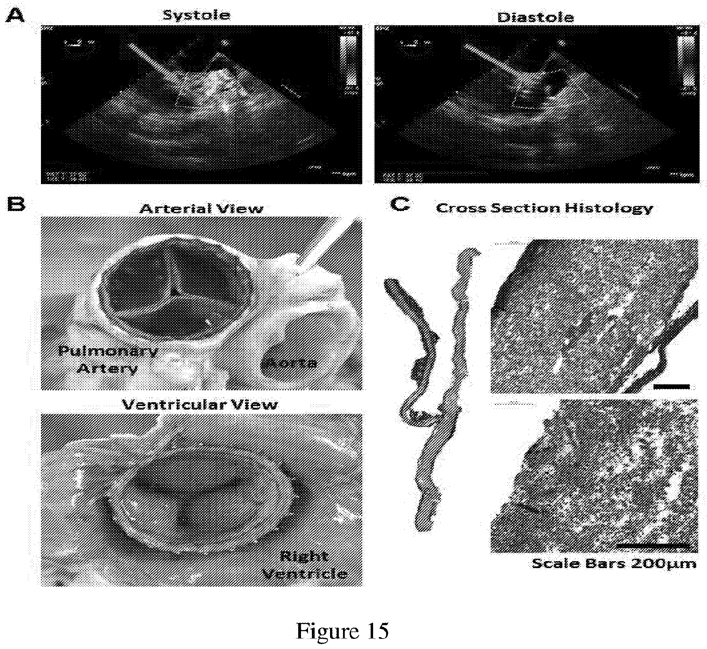

Acute trials (15 hours) to assess the *in vivo* functionality of the engineered valves, *i.e.,* semilunar valves, with integral leaflets fabricated as described herein were performed using a standard ovine pulmonary valve implantation model. Semilunar valves were formed according to the method described above with respect to Figure 10. Each valve was attached to an outer outer shape-memory metal stent (*i.e.,* a nitinol stent) using anchor sutures, and then implanted to replace an ovine pulmonary valve. The results appear in Figures 15A-15C. At acute time points, the valve leaflets are opening and closing once implanted, with minimal transvalvular pressures (0-2 mmHg) during systole (Figure 15A, left-hand image), demonstrating no stenosis or resistance to outward flow. During diastole (Figure 15A, righthand image), Doppler imaging confirms full valve closure with only minor closing volume during the beginning of diastole. After explantation, there was no major clotting or loss of scaffold structure (Figure 15B), and there was full wetting of the scaffold. Furthermore, histological analysis revealed complete native cell penetration (e.g., neutrophils at the 15 hour time point) (Figure 15C), indicating the promotion of endogenous remodeling.

### IV. Method of Making Engineered Tubular Structures with Embedded Device

Another embodiment of the present invention is an engineered tubular structure with an embedded device, such as a stent, and a method of making such an engineered tubular structure. For example, a first portion of polymeric fibers including micron, submicron or nanometer dimension polymer fibers are collected on an outer surface of a mandrel. In some embodiments, the polymeric fibers are formed by ejecting or flinging polymer from a reservoir onto a rotating mandrel in the path of the ejected or flung polymeric fibers in a manner similar to the deposition of the polymeric fibers onto the combined mandrel for the valve with embedded stent, which is describe above. After deposition of the first portion of the polymeric fibers onto the mandrel, a device such as a stent is positioned on the mandrel over the first portion of the polymer fibers. The device may be secured to the mandrel by any appropriate means (*e.g.,* by screws, clamps, clips, bands, *etc.*)*.* A second portion of the polymeric fibers is collected on the mandrel over the first portion and over the device forming a tubular wall in which the device is embedded. The mandrel could be withdrawn from within the tubular wall leaving the tubular wall with the stent embedded therein.

One of skill in the art would appreciate that the methods described above with respect to making a valve with an embedded stent may be applied to make an engineered tubular structure with an embedded stent. One of skill in the art would appreciate that the teaching and disclosure above regarding the tubular wall portion of the valve also apply to the tubular wall of the engineered tubular structure. Further, the skilled artisan, in view of the present disclosure, would recognize an engineered tubular structure may include multiple embedded stents, one or more embedded medical devices or elements other than stents, or a combination of different types of embedded medical devices or elements.

The structure of the resulting engineered tubular structure may be configured to form a polymeric fiber scaffold for cellular ingrowth. In some embodiments, the resulting valve could be seeded with cells to form a tissue-engineered tubular structure (described below).

### V. Engineered Polymeric Fiber Sheets

Some embodiments of the invention include an engineered polymeric fiber sheet. For example, in some embodiments, after an engineered tubular structure is formed as described above, the tubular structure may be cut and flattened to form a sheet. In other embodiments, polymeric fibers including micron, submicron and/or nanometer dimension fibers are be collected on a flat collection device, as opposed to on a rotating mandrel. The polymeric fibers are formed by ejecting or flinging a polymer from reservoir (*e.g*., as described above with respect to Figures 1A-1C). Control of movement of the collection device during collection of the polymeric fibers may be used to control an orientation of the polymeric fibers. For example, if the polymeric fibers are collected on a rotating mandrel, an orientation of the mandrel rotation axis relative to the deposition rotation axis and/or a rate of translation along the mandrel rotation axis (or parallel to the deposition rotation axis) may be used to control collected fiber orientation. As another example, if the polymeric fibers are collected on a flat sheet, a rate of translation of the flat sheet laterally, an orientation of the sheet and/or rotation of the sheet may be used to control collected fiber orientation.

The engineered polymeric fiber sheet may include one or more embedded elements. The one or more embedded elements include, but are not limited to metal mesh structures, support meshes, pressure transducers, strain transducers, time-released therapeutic agents (*e.g.,* in capsules or pouches); flow sensors; actuators; optical tracers (e*.g.* in capsules or pouches, which could be activated or released for imaging). The one or more elements may be embedded in the resulting engineered polymeric fiber sheet by collecting a first portion of polymeric fibers on the collection device, positioning at least some of the elements on the collection device over the first portion of the polymeric fibers, and then depositing a second portion of the polymeric fibers over the elements. In some embodiments, all elements are embedded in a same layer of the engineered polymeric fiber sheet. In other embodiments, elements are embedded in different layers of the resulting engineered polymeric sheet.

The structure of the engineered polymeric fiber sheet may be configured to form a polymeric fiber scaffold for cellular ingrowth. In some embodiments, the resulting valve could be seeded with cells to form a tissue-engineered sheet.

### VI. Methods for Generating Engineered Tissues

In certain embodiments, the engineered valves, tubular structures, and sheets may comprise an engineered tissue which is fabricated by seeding cells onto the polymeric fibers of a valve, tubular structure, or sheet, and culturing the cells to form a functional tissue. For example, in some embodiments, an engineered valve fabricated as described herein is seeded with cells which are cultured under suitable conditions to form a functional tissue prior to implantation into a subject as a replacement valve.

In other embodiments, the engineered valves (and tubular structures and sheets) fabricated as described herein are not seeded with cells that are cultured to form a functional tissue prior to implantation into a subject as a replacement valve, thereby significantly simplifying the fabrication process of a replacement valve and significantly reducing the costs for fabrication of a replacement valve. In such embodiments, native cells migrate into the engineered valve when implanted in a subject, grow, and form a functional tissue.

Accordingly, in some embodiments of the methods of the invention , a valve, tubular structure and/or sheet, as described above, is seeded with cells and cultured in an incubator under physiologic conditions (e.g., at 37°C) until the cells form an engineered tissue.

Any appropriate cell culture method may be used. The seeding density of the cells will vary depending on the cell size and cell type, but can easily be determined by methods known in the art. In one embodiment, cardiac myocytes are seeded at a density of between about 1 × 10⁵ to about 6 × 10⁵ cells/cm², or at a density of about 1 × 10⁴, about 2 × 10⁴, about 3 × 10⁴, about 4 × 10⁴, about 5 × 10⁴, about 6 × 10⁴, about 7 × 10⁴, about 8 × 10⁴, about 9 × 10⁴, about 1 × 10⁵, about 1.5 × 10⁵, about 2 × 10⁵, about 2.5 × 10⁵, about 3 × 10⁵, about 3.5 × 10⁵, about 4 × 10⁵, about 4.5 × 10⁵, about 5 × 10⁵, about 5.5 × 10⁵, about 6 × 10⁵, about 6.5 × 10⁵, about 7 × 10⁵, about 7.5 × 10⁵, about 8 × 10⁵, about 8.5 × 10⁵, about 9 × 10⁵, about 9.5 × 10⁵, about 1 × 10⁶, about 1.5 × 10⁶, about 2 × 10⁶, about 2.5 × 10⁶, about 3 × 10⁶, about 3.5 × 10⁶, about 4 × 10⁶, about 4.5 × 10⁶, about 5 × 10⁶, about 5.5 × 10⁶, about 6 × 10⁶, about 6.5 × 10⁶, about 7 × 10⁶, about 7.5 × 10⁶, about 8 × 10⁶, about 8.5 × 10⁶, about 9 × 10⁶, or about 9.5 × 10⁶. Values and ranges intermediate to the above-recited values and ranges are also contemplated by the present invention.

In some embodiments, a valve, tubular structure and/or sheet is contacted with living cells during the fabrication process such that a structure populated with cells or fibers surrounded (partially or totally) with cells are produced. The valve, tubular structure and/or sheet may also be contacted with additional agents, such as proteins, nucleotides, lipids, drugs, pharmaceutically active agents, biocidal and antimicrobial agents during the fabrication process such that functional micron, submicron or nanometer dimension polymeric fibers are produced which contain these agents. For example, fibers comprising living cells may be fabricated by providing a polymer and living cells in a solution of cell media at a concentration that maintains cell viability.

Suitable cells for use in the invention may be normal cells, abnormal cells (*e.g.,* those derived from a diseased tissue, or those that are physically or genetically altered to achieve an abnormal or pathological phenotype or function), normal or diseased muscle cells derived from embryonic stem cells or induced pluripotent stem cells. Suitable cells include umbilical endothelial cells, vascular endothelial cells, mesenchymal stem cells, primary valve harvest endothelial/interstitial cells, and cardiomycocytes. Such cells may be seeded on the scaffold including leaflets and cultured to form a functional tissue, such as a functional valve tissue.

Cells for seeding can be cultured *in vitro,* derived from a natural source, genetically engineered, or produced by any other means. Any natural source of prokaryotic or eukaryotic cells may be used. Embodiments in which the a valve, tubular structure and/or sheet is implanted in an organism can use cells from the recipient, cells from a conspecific donor or a donor from a different species, or bacteria or microbial cells.

The term "progenitor cell" is used herein to refer to cells that have a cellular phenotype that is more primitive (*e.g*., is at an earlier step along a developmental pathway or progression than is a fully differentiated cell) relative to a cell which it can give rise to by differentiation. Often, progenitor cells also have significant or very high proliferative potential. Progenitor cells can give rise to multiple distinct differentiated cell types or to a single differentiated cell type, depending on the developmental pathway and on the environment in which the cells develop and differentiate.

The term "progenitor cell" is used herein synonymously with "stem cell."

The term "stem cell" as used herein, refers to an undifferentiated cell which is capable of proliferation and giving rise to more progenitor cells having the ability to generate a large number of mother cells that can in turn give rise to differentiated, or differentiable daughter cells. The daughter cells themselves can be induced to proliferate and produce progeny that subsequently differentiate into one or more mature cell types, while also retaining one or more cells with parental developmental potential. The term "stem cell" refers to a subset of progenitors that have the capacity or potential, under particular circumstances, to differentiate to a more specialized or differentiated phenotype, and which retains the capacity, under certain circumstances, to proliferate without substantially differentiating. In one embodiment, the term stem cell refers generally to a naturally occurring mother cell whose descendants (progeny) specialize, often in different directions, by differentiation, *e.g*., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. While each of these multipotent cells may be considered stem cells, the range of cell types each can give rise to may vary considerably. Some differentiated cells also have the capacity to give rise to cells of greater developmental potential. Such capacity may be natural or may be induced artificially upon treatment with various factors. In many biological instances, stem cells are also "multipotent" because they can produce progeny of more than one distinct cell type, but this is not required for "stem-ness." Self-renewal is the other classical part of the stem cell definition. In theory, self-renewal can occur by either of two major mechanisms. Stem cells may divide asymmetrically, with one daughter retaining the stem state and the other daughter expressing some distinct other specific function and phenotype. Alternatively, some of the stem cells in a population can divide symmetrically into two stems, thus maintaining some stem cells in the population as a whole, while other cells in the population give rise to differentiated progeny only. Formally, it is possible that cells that begin as stem cells might proceed toward a differentiated phenotype, but then "reverse" and re-express the stem cell phenotype, a term often referred to as "dedifferentiation" or "reprogramming" or "retrodifferentiation".

The term "embryonic stem cell" is used to refer to the pluripotent stem cells of the inner cell mass of the embryonic blastocyst (see US Patent Nos. 5,843,780, 6,200,806.

Such cells can similarly be obtained from the inner cell mass of blastocysts derived from somatic cell nuclear transfer (see, for example, US Patent Nos. 5,945,577, 5,994,619, 6,235,970. The distinguishing characteristics of an embryonic stem cell define an embryonic stem cell phenotype. Accordingly, a cell has the phenotype of an embryonic stem cell if it possesses one or more of the unique characteristics of an embryonic stem cell such that that cell can be distinguished from other cells. Exemplary distinguishing embryonic stem cell characteristics include, without limitation, gene expression profile, proliferative capacity, differentiation capacity, karyotype, responsiveness to particular culture conditions, and the like.

The term "adult stem cell" or "ASC" is used to refer to any multipotent stem cell derived from non- embryonic tissue, including fetal, juvenile, and adult tissue. Stem cells have been isolated from a wide variety of adult tissues including blood, bone marrow, brain, olfactory epithelium, skin, pancreas, skeletal muscle, and cardiac muscle. Each of these stem cells can be characterized based on gene expression, factor responsiveness, and morphology in culture. Exemplary adult stem cells include neural stem cells, neural crest stem cells, mesenchymal stem cells, hematopoietic stem cells, and pancreatic stem cells.

In one embodiment, progenitor cells suitable for use in the claimed devices and methods are Committed Ventricular Progenitor (CVP) cells as described in PCT Application No. WO 2010/042856, entitled "Tissue Engineered Mycocardium and Methods of Productions and Uses Thereof', filed October 9, 2009.

### VII. Use of Engineered Valves, Engineered Tubular Structures, and Fiber Sheets of the Invention

The engineered valves, tubular structures and/or sheets of the invention may be used *in vitro* or *in vivo.* The engineered valves, tubular structures and/or sheets described herein have various applications. For example, they may be used as engineered tissues and/or for implantation, repair and replacement of biological tissues and organs.

For example, the engineered valves of the invention may be used as replacement valves in any subjects having defective or weakened tricuspid valves, mitral valves, semilunar valves and/or venous valve.

For *in vivo* applications, the exact size and shape of the valves are species and patient specific. For example, pediatric patients may require smaller valves than adult patients. Pediatric aortic diameters can range from about 10 mm -20 mm (*i.e.,* the diameter of the aortic annuls). Adults can have an aortic annulus ranging from 20-35 mm in diameter.

One benefit of the valves of the present invention is that they can be fabricated and custom-sized to fit the subject.

Additionally, the valves of the present invention do not require that cells be seeded and cultured prior to implantation into a subject, as the valves are configured to permit native cells to populate the valves.

In addition, in embodiments in which cells are seeded and cultured prior to implantation of the valve into a subject, the use of autologous cells to seed the valves permits the subject to forego immunosuppressive therapy. Moreover, the engineered tissues will be integrated into the natural tissue as cells from the subject will integrate into the polymeric scaffold of the valves and remodel the scaffold.

Any suitable means for accessing the subject's heart and attaching the devices may be used, such as thoracic surgery or transmyocardial catheter delivery.

Engineered valves and engineered tubular structures may also be employed in blood vessel repair or replacement (*e.g*., aortic aneurysm repair, bypass grafts for cardiac surgery, major artery/venous repair and/or replacement, *etc*.). Engineered valves and engineered tubular structures with an anisotropic orientation of polymeric fibers may be particular useful for blood vessel repair or replacement.

However, the applications of engineered valves and engineered tubular structures are not limited to blood vessels. For example, airway bronchi, esophagus, intestinal tubes can all be created using the methods described herein. Also, the engineered tubular structures can act as replacements for extracted or diseased tissues in these tubular regions. For example, scarred or ruptured intestine can be removed and replaced with an engineered tubular structure instead of removing the tissue and suturing the ends of native tissue together. Likewise, damaged esophagus (*e.g*., due to cancer or acid reflux) can be removed and replaced with an engineered fiber structure.

Furthermore, because the methods here provide controlled fiber orientation and can be used with differently shaped mandrels, curvatures and diameters specific to a particular patient can be achieved in a "customized tissue engineering" approach.

The engineered polymeric fiber sheets described herein have various applications. For example, they may be used as engineered tissues and/or for implantation, repair and replacement of biological tissues and organs. Engineered polymeric fiber sheets with an anisotropic orientation of polymeric fibers may be particular useful for repair or replacement of tissues that exhibit significant anisotropy (*e.g*., muscle tissues).

Embodiments of engineered polymeric fiber sheets may be employed for applications including skin-wound healing, cardiac patches, hernia patches, *etc.*

## Claims

1. An engineered valve (300, 400) comprising:
a tubular wall (310, 410) comprising micron, submicron or nanometer dimension polymer fibers defining a shape of the tubular wall (310, 410), the tubular wall (310, 410) having an inner surface, a first portion (270), and a second portion (274);
one or more leaflets (320a, 320b) extending from, and integral with, the inner surface of the tubular wall (310, 410), the one or more leaflets (320a, 320b) each comprising micron, submicron or nanometer dimension polymer fibers defining the shape of the corresponding leaflet (320a, 320b);
wherein the one or more leaflets (320a, 320b) are configured for one-way fluid flow through the first portion (270) of the tubular wall (310, 410), past the one or more leaflets (320a, 320b), and into the second portion (272) of the tubular wall (310, 410), and wherein the second portion of the tubular wall (272) includes all of the tubular wall (310, 410) downstream of the one or more leaflets (320a, 320b);
wherein, for each of the one or more leaflets (320a, 320b), at least some of the micron, submicron or nanometer dimension polymer fibers (240) forming a part of the leaflet (320a, 320b) interpenetrate with at least some of the micron, submicron or nanometer dimension polymer fibers forming a part of the first portion (270) of the tubular wall (310, 410); and
wherein the micron, submicron or nanometer dimension polymer fibers of the tubular wall (310, 410) and the micron, submicron or nanometer dimension polymer fibers (240) of the one or more leaflets (320a, 320b) are configured to form a polymeric fiber scaffold for cellular ingrowth into the tubular wall (310, 410) and the one or more leaflets (320a, 320b).

2. The engineered valve (300, 400) of claim 1, wherein
(a) at least some of the micron, submicron or nanometer dimension polymer fibers of the one or more leaflets (320a, 320b) interpenetrate with at least some of the micron, submicron or nanometer dimension polymer fibers of the inner surface of the tubular wall (310, 410); or
(b) the micron, submicron or nanometer dimension polymer fibers of the one or more leaflets (320a, 320b) interpenetrate with the micron, submicron or nanometer dimension polymer fibers of the inner surface of the tubular wall (310, 410) where the one or more leaflets (320a, 320b) meet the inner surface of the tubular wall (310, 410); or
(c) the leaflets (320a, 320b) are also integral with the outer surface of the tubular wall (310, 410); or
(d) the tubular wall (310, 410) further comprises a stent (276) embedded in the micron, submicron or nanometer dimension polymer fibers; or
(e) the micron, submicron or nanometer dimension polymer fibers each have a diameter of between about 0.5 µm and about 1.5 µm.

3. The engineered valve (300, 400) of claim 1, wherein the valve (300, 400) is formed by:
forming a first portion of the polymeric fibers by ejecting or flinging a polymer from a reservoir (102) onto a first mandrel (200);
collecting the first portion of formed polymeric fibers (240) on an outer surface (202) of the first mandrel (200) to at least partially form the first portion (270) of the tubular wall (310, 410) and the one or more leaflets (320a, 320b) connected to, and integral with, an inner surface of the first portion (270) of the tubular wall (310, 410), the formed polymeric fibers including micron, submicron, and/or nanometer dimension polymeric fibers, the outer surface (202) of the first mandrel including:
a first tubular forming portion (204) having a shape corresponding to the inner surface of the first portion (270) of the tubular wall (310, 410), and
one or more leaflet forming portions (206a, 206b), each having a shape corresponding to a first surface (322a) of a corresponding one of the one or more leaflets (320a, 320b) of the resulting valve (300, 400);
positioning a second mandrel (220) with respect to the first mandrel (200) having the collected first portion of polymer fibers (240) thereon, the second mandrel (220) having an outer surface (222) including a second tubular forming portion (224) having a shape corresponding to an inner surface of a second portion (272) of the resulting tubular wall (310, 410), the first mandrel (200) and second mandrel (220) together forming a combined mandrel (250);
forming a second portion (254) of the polymeric fibers by ejecting or flinging the polymer from the reservoir (102) onto the combined mandrel (250); and
collecting the second portion (254) of the polymeric fibers on an outside surface of the combined mandrel (250) thereby forming, at least, the second portion (272) of the tubular wall (310, 410).

4. A method of making a valve (300, 400) including a tubular wall (310, 410) and one or more leaflets (320a 320b) integral with and extending from an inner surface of the tubular wall (310, 410), the method comprising:
collecting a first portion of formed polymeric fibers (240) on an outer surface (202) of a first mandrel (200) to at least partially form a first portion (270) of a tubular wall (310, 410) and one or more leaflets (320a, 320b) connected to, and integral with, an inner surface of the first portion (270) of the tubular wall (310, 410), the formed polymeric fibers including micron, submicron, and/or nanometer dimension polymeric fibers, wherein, for each of the one or more leaflets (320a, 320b), at least some of the micron, submicron or nanometer dimension polymer fibers forming a part of the leaflet (320a, 320b) interpenetrate with at least some of the micron, submicron or nanometer dimension polymer fibers forming a part of the upstream first portion (270) of the tubular wall (310, 410), the outer surface (202) of the first mandrel including:
a first tubular forming portion (204) having a shape corresponding to the inner surface of the first portion (270) of the tubular wall (310, 410), and
one or more leaflet forming portions (206a, 206b), each having a shape corresponding to a first surface (322a) of a corresponding one of the one or more leaflets (320a, 320b) of the resulting valve (300, 400);
positioning a second mandrel (220) with respect to the first mandrel (200) having the collected first portion of polymer fibers (240) thereon, the second mandrel (220) having an outer surface (222) including a second tubular forming portion (224) having a shape corresponding to an inner surface of a second portion (272) of the resulting tubular wall (310, 410), the first mandrel (200) and second mandrel (220) together forming a combined mandrel (250); and
collecting a second portion (254) of the formed polymeric fibers on an outside surface of the combined mandrel (250) thereby forming, at least, the second portion (272) of the tubular wall (310, 410), wherein the second portion (272) of the resulting tubular wall is the portion of the resulting tubular wall that is downstream of the one or more leaflet (320a, 320b) during one-way fluid flow through the resulting valve (300, 400);
wherein the micron, submicron or nanometer dimension polymer fibers of the tubular wall (310, 410) and the micron, submicron or nanometer dimension polymer fibers of the one or more leaflets (320a, 320b) are configured to form a polymeric fiber scaffold for cellular ingrowth into the tubular wall (310, 410) and the one or more leaflets (320a, 320b).

5. The method of claim 4, further comprising:
forming the first portion of the polymeric fibers (240) by ejecting or flinging a polymer from a reservoir (102) onto the first mandrel (200); and
forming the second portion (254) of the polymeric fibers by ejecting or flinging the polymer from the reservoir (102) onto the combined mandrel (250); optionally wherein (a) the polymeric fibers are ejected from the reservoir (102) and the reservoir (102) is rotating at a speed of between 20,000 and 60,000 rpm; or (b) the reservoir (102) is rotating at a speed of between 25,000 and 35,000 rpm,
optionally, wherein the reservoir (102) is free of a high voltage electrical field during formation of the first portion of the polymeric fibers (240) and during formation of the second portion of the polymer fibers (254).

6. The method of claim 5, wherein the polymeric fibers are ejected from the reservoir (102) and the reservoir (102) is rotating at a speed of between 20,000 and 60,000 rpm;
wherein collecting the first portion of the polymeric fibers (240) comprises rotating the first mandrel (200) about a mandrel rotation axis (242) in a path of the ejected or flung polymer fibers; and
wherein collecting the second portion (254) of the polymeric fibers comprises rotating the combined mandrel (250) about the mandrel rotation axis (242) in a path of the ejected or flung polymer fibers; optionally,
a) wherein the first mandrel (200) is rotated about the mandrel rotation axis (242) at a rotation speed between 1,000 and 6,000 rpm during collection of the first portion of the polymeric fibers (240) and the combined mandrel (250) is rotated about the mandrel rotation axis (242) at a rotation speed between 1,000 and 6,000 rpm during collection of the second portion (254) of the polymeric fibers; or
b) wherein the first mandrel (200) is rotated about the mandrel rotation axis (242) at a rotation speed between 2,000 and 4,000 rpm during collection of the first portion of the polymeric fibers (240) and the combined mandrel (250) is rotated about the mandrel rotation axis (242) at a rotation speed between 2,000 and 4,000 rpm during collection of the second portion (254) of the polymeric fibers.

7. The method of claim 6, further comprising linearly translating the rotating combined mandrel (250) during collection of the second portion (254) of the polymeric fibers.

8. The method of claim 4, wherein a surface of the second mandrel (220) includes one or more leaflet forming portions, each having a shape corresponding to a second surface of a corresponding one of the one or more leaflets (320a, 320b) in the resulting valve (300, 400); optionally wherein the at least one first leaflet forming portion (206a, 206b) of the first mandrel (200) has a concave shape, and the at least one leaflet forming portion of the second mandrel (220) has a convex shape (228a, 228b, 228c).

9. The method as claimed in claim 4, wherein the first portion of the polymeric fibers (240) is disposed between the first mandrel (200) and the second mandrel (220) and on the first tubular forming portion (204) of the outside surface (202) of the first mandrel (200) when the second mandrel (220) is positioned with respect to the first mandrel (200) to form the combined mandrel (250);
optionally, wherein the combined mandrel (250) forms a crevasse between the first mandrel (200) and the second mandrel (220) with the crevasse extending radially inward from the outer surface of the combined mandrel (250);
optionally, wherein the first portion of the polymeric fibers (240) is disposed within the crevasse and extends at least to a boundary between the crevasse and first tubular forming portion (204), such that when the second portion (254) of the polymeric fibers is collected on the combined mandrel (250), the second portion (254) of polymeric fibers integrates with the first portion of polymeric fibers (240).

10. The method of claim 4, wherein positioning the second mandrel (220) with respect to the first mandrel (200) comprises engaging at least one first engagement portion (208a, 208b, 208c) of the first mandrel (200) with a corresponding at least one second engagement portion (228a) of the second mandrel (220); optionally wherein the at least one first engagement portion (208a, 208b, 208c) comprises at least one protrusion, and the at least one second engagement portion (228a) comprises at least one recess configured to receive the at least one protrusion (208a, 208b, 208c).

11. The method of claim 4, further comprising:
(a) positioning a stent (276) over the second portion (254) of the polymeric fibers collected on the outside surface of the combined mandrel (250); and
collecting a third portion (280) of the polymeric fibers on an outside surface of the stent (276) to form the tubular wall (310, 410) with the stent (276) embedded therein; or
(b) seeding cells onto the polymeric fibers of the tubular wall (310, 410).

12. The method of claim 4, wherein the tubular wall (310, 410) includes a first end (301a) and a second end (301b) with the one or more leaflets (320a, 320b) disposed between the first end (301a) and the second end (301b), and wherein the method further comprises:
withdrawing the first mandrel (200) from within the tubular wall (310, 410) via the first end (301a); and
withdrawing the second mandrel (220) from within the tubular wall (310, 410) via the second end (301b).

13. The method of claim 4, wherein each formed polymeric fiber has a diameter of between about 0.5 µm and about 1.5 µm, or wherein an average diameter of the formed polymeric fibers is between about 0.75 µm and about 1.25 µm.

14. The method of claim 4, wherein the first mandrel (200) includes at least two leaflet forming portions (206a, 206b), wherein collecting the first portion (270) of the formed polymeric fibers (240) on the outer surface (202) of the first mandrel (200) at least partially forms the first portion (270) of the tubular wall (310, 410) and each of two or more leaflets (320a, 320b) connected to, and integral with, the inner surface of the first portion (270) of the tubular wall (310, 410).

## Patentansprüche

1. Technisches Ventil (300, 400), welches umfasst:
eine röhrenförmige Wand (310, 410), die Polymerfasern im Mikrometer-, Submikrometer- oder Nanometerbereich umfasst, die eine Form der röhrenförmigen Wand (310, 410) definieren, wobei die röhrenförmige Wand (310, 410) eine Innenfläche, einen ersten Abschnitt (270) und einen zweiten Abschnitt (274) aufweist;
ein oder mehrere Blättchen (320a, 320b), die sich von der Innenfläche der röhrenförmigen Wand (310, 410) erstrecken und mit dieser einstückig sind, wobei das eine oder die mehreren Blättchen (320a, 320b) jeweils Polymerfasern im Mikrometer-, Submikrometer- oder Nanometerbereich umfasst, die die Form des entsprechenden Blättchens (320a, 320b) definieren;
wobei das eine oder die mehreren Blättchen (320a, 320b) für eine Einweg-Fluidströmung durch den ersten Abschnitt (270) der röhrenförmigen Wand (310, 410), an dem einen oder den mehreren Blättchen (320a, 320b) vorbei und in den zweiten Abschnitt (272) der röhrenförmigen Wand (310, 410) hinein ausgestaltet ist, und wobei der zweite Abschnitt der röhrenförmigen Wand (272) die gesamte röhrenförmige Wand (310, 410) stromabwärts von dem einen oder den mehreren Blättchen (320a, 320b) umfasst;
wobei für jedes der einen oder mehreren Blättchen (320a, 320b) mindestens einige der Polymerfasern (240) im Mikrometer-, Submikrometer- oder Nanometerbereich, die einen Teil des Blättchens (320a, 320b) ausbilden, mit mindestens einigen der Polymerfasern im Mikrometer-, Submikrometer- oder Nanometerbereich, die einen Teil des ersten Abschnitts (270) der röhrenförmigen Wand (310, 410) ausbilden, interpenetrieren; und
wobei die Polymerfasern im Mikrometer-, Submikrometer- oder Nanometerbereich der röhrenförmigen Wand (310, 410) und die Polymerfasern im Mikrometer-, Submikrometer- oder Nanometerbereich (240) des einen oder der mehreren Blättchen (320a, 320b) ausgestaltet sind, ein Gerüst aus Polymerfasern für das Einwachsen von Zellen in die röhrenförmige Wand (310, 410) und in das eine oder die mehreren Blättchen (320a, 320b) auszubilden.

2. Technisches Ventil (300, 400) nach Anspruch 1, wobei
(a) mindestens einige der Polymerfasern im Mikrometer-, Submikrometer- oder Nanometerbereich der einen oder mehreren Blättchen (320a, 320b) mit mindestens einigen der Polymerfasern im Mikrometer-, Submikrometer- oder Nanometerbereich der Innenfläche der röhrenförmigen Wand (310, 410) interpenetrieren; oder
(b) die Polymerfasern im Mikrometer-, Submikrometer- oder Nanometerbereich der einen oder mehreren Blättchen (320a, 320b) mit den Polymerfasern im Mikrometer-, Submikrometer- oder Nanometerbereich der Innenfläche der röhrenförmigen Wand (310, 410) dort interpenetrieren, wo die eine oder mehreren Blättchen (320a, 320b) auf die Innenfläche der röhrenförmigen Wand (310, 410) treffen; oder
(c) die Blättchen (320a, 320b) auch mit der Außenfläche der röhrenförmigen Wand (310, 410) einstückig sind; oder
(d) die röhrenförmige Wand (310, 410) ferner einen Stent (276) umfasst, der in die Polymerfasern im Mikrometer-, Submikrometer- oder Nanometerbereich eingebettet ist; oder
(e) die Polymerfasern im Mikrometer-, Submikrometer- oder Nanometerbereich jeweils einen Durchmesser zwischen etwa 0,5 µm und etwa 1,5 µm aufweisen.

3. Technisches Ventil (300, 400) nach Anspruch 1, wobei das Ventil (300, 400) ausgebildet wird durch:
Ausbilden eines ersten Abschnitts der Polymerfasern durch Ausstoßen oder Schleudern eines Polymers aus einem Vorratsbehälter (102) auf einen ersten Dorn (200);
Sammeln des ersten Abschnitts der gebildeten Polymerfasern (240) auf einer Außenfläche (202) des ersten Dorns (200), um zumindest teilweise den ersten Teil (270) der röhrenförmigen Wand (310, 410) und das eine oder die mehreren Blättchen (320a, 320b) auszubilden, die mit einer Innenfläche des ersten Abschnitts (270) der röhrenförmigen Wand (310, 410) verbunden sind und einstückig damit vorliegen, wobei die gebildeten Polymerfasern solche Polymerfasern im Mikrometer-, Submikrometer- und/oder Nanometerbereich umfassen, wobei die Außenfläche (202) des ersten Dorns umfasst:
einen ersten röhrenförmigen Formungsabschnitt (204) mit einer Form, die der Innenfläche des ersten Abschnitts (270) der röhrenförmigen Wand (310, 410) entspricht, und
einen oder mehrere Blättchen ausbildende Abschnitte (206a, 206b), die jeweils eine Form aufweisen, die einer ersten Oberfläche (322a) eines entsprechenden der einen oder mehreren Blättchen (320a, 320b) des entstehenden Ventils (300, 400) entspricht;
Anordnen eines zweiten Dorns (220) in Bezug auf den ersten Dorn (200), auf dem sich der gesammelte erste Abschnitt von Polymerfasern (240) befindet, wobei der zweite Dorn (220) eine Außenfläche (222) aufweist, die einen zweiten röhrenförmigen Formungsabschnitt (224) mit einer Form enthält, die einer Innenfläche eines zweiten Abschnitts (272) der entstehenden röhrenförmigen Wand (310, 410) entspricht, wobei der erste Dorn (200) und der zweite Dorn (220) zusammen einen kombinierten Dorn (250) ausbilden;
Ausbilden eines zweiten Abschnitts (254) der Polymerfasern durch Ausstoßen oder Schleudern des Polymers aus dem Vorratsbehälter (102) auf den kombinierten Dorn (250); und
Sammeln des zweiten Abschnitts (254) der Polymerfasern auf einer Außenfläche des kombinierten Dorns (250), wodurch mindestens der zweite Abschnitt (272) der röhrenförmigen Wand (310, 410) ausgebildet wird.

4. Verfahren zur Herstellung eines Ventils (300, 400) mit einer röhrenförmigen Wand (310, 410) und einem oder mehreren Blättchen (320a, 320b), die einstückig mit einer Innenfläche der röhrenförmigen Wand (310, 410) ausgebildet sind und sich von dieser erstrecken, wobei das Verfahren umfasst:
Sammeln eines ersten Abschnitts geformter Polymerfasern (240) auf einer Außenfläche (202) eines ersten Dorns (200), um zumindest teilweise einen ersten Abschnitt (270) einer röhrenförmigen Wand (310, 410) und ein oder mehrere Blättchen (320a, 320b) auszubilden, die mit einer Innenfläche des ersten Abschnitts (270) der röhrenförmigen Wand (310, 410) verbunden sind und einstückig damit vorliegen, worin die geformten Polymerfasern solche Polymerfasern im Mikrometer-, Submikrometer- und/oder Nanometerbereich umfassen, wobei für jedes der einen oder mehreren Blättchen (320a, 320b) mindestens einige der Polymerfasern im Mikrometer-, Submikrometer- und/oder Nanometerbereich, die einen Teil des Blättchens (320a, 320b) ausbilden, mit mindestens einigen der Polymerfasern im Mikrometer-, Submikrometer- und/oder Nanometerbereich, die einen Teil des stromaufwärts gelegenen ersten Abschnitts (270) der röhrenförmigen Wand (310, 410) ausbilden, interpenetrieren, wobei die Außenfläche (202) des ersten Dorns umfasst:
einen ersten röhrenförmigen Formungsabschnitt (204), der eine Form aufweist, die der Innenfläche des ersten Abschnitts (270) der röhrenförmigen Wand (310, 410) entspricht, und
einen oder mehrere Blättchen ausbildende Abschnitte (206a, 206b), die jeweils eine Form aufweisen, die einer ersten Oberfläche (322a) eines entsprechenden der einen oder mehreren Blättchen (320a, 320b) des entstehenden Ventils (300, 400) entspricht;
Anordnen eines zweiten Dorns (220) in Bezug auf den ersten Dorn (200), auf dem sich der gesammelte erste Abschnitt von Polymerfasern (240) befindet, wobei der zweite Dorn (220) eine Außenfläche (222) aufweist, die einen zweiten röhrenförmigen Formungsabschnitt (224) mit einer Form enthält, die einer Innenfläche eines zweiten Abschnitts (272) der entstehenden röhrenförmigen Wand (310, 410) entspricht, wobei der erste Dorn (200) und der zweite Dorn (220) zusammen einen kombinierten Dorn (250) ausbilden; und
Sammeln eines zweiten Abschnitts (254) der gebildeten Polymerfasern auf einer Außenfläche des kombinierten Dorns (250), wodurch zumindest der zweite Abschnitt (272) der röhrenförmigen Wand (310, 410) ausgebildet wird, wobei der zweite Abschnitt (272) der entstehenden röhrenförmigen Wand der Abschnitt der entstehenden röhrenförmigen Wand ist, der sich stromabwärts von dem einen oder mehreren Blättchen (320a, 320b) während des Einweg-Fluidflusses durch das entstandene Ventil (300, 400) befindet;
wobei die Polymerfasern im Mikrometer-, Submikrometer- oder Nanometerbereich der röhrenförmigen Wand (310, 410) und die Polymerfasern im Mikrometer-, Submikrometer- oder Nanometerbereich des einen oder der mehreren Blättchen (320a, 320b) ausgestaltet sind, ein Gerüst aus Polymerfasern für das Einwachsen von Zellen in die röhrenförmige Wand (310, 410) und das eine oder die mehreren Blättchen (320a, 320b) auszubilden.

5. Verfahren nach Anspruch 4, welches ferner umfasst:
Ausbilden des ersten Abschnitts der Polymerfasern (240) durch Ausstoßen oder Schleudern eines Polymers aus einem Vorratsbehälter (102) auf den ersten Dorn (200); und
Ausbilden des zweiten Abschnitts (254) der Polymerfasern durch Ausstoßen oder Schleudern des Polymers aus dem Vorratsbehälter (102) auf den kombinierten Dorn (250); wobei gegebenenfalls (a) die Polymerfasern aus dem Vorratsbehälter (102) ausgestoßen werden und der Vorratsbehälter (102) mit einer Geschwindigkeit zwischen 20.000 und 60.000 U/min rotiert; oder (b) der Vorratsbehälter (102) mit einer Geschwindigkeit zwischen 25.000 und 35.000 U/min rotiert,
wahlweise, wobei der Vorratsbehälter (102) während der Bildung des ersten Abschnitts der Polymerfasern (240) und während der Bildung des zweiten Abschnitts der Polymerfasern (254) frei von einem elektrischen Hochspannungsfeld ist.

6. Verfahren nach Anspruch 5, wobei die Polymerfasern aus dem Vorratsbehälter (102) ausgestoßen werden und der Vorratsbehälter (102) mit einer Geschwindigkeit zwischen 20.000 und 60.000 U/min rotiert;
wobei das Sammeln des ersten Abschnitts der Polymerfasern (240) das Drehen des ersten Dorns (200) um eine Dornrotationsachse (242) in einer Bahn der ausgeworfenen oder geschleuderten Polymerfasern umfasst; und
wobei das Sammeln des zweiten Abschnitts (254) der Polymerfasern das Drehen des kombinierten Dorns (250) um die Dorndrehachse (242) in einer Bahn der ausgeworfenen oder geschleuderten Polymerfasern umfasst; wahlweise,
a) wobei der erste Dorn (200) während des Sammelns des ersten Abschnitts der Polymerfasern (240) mit einer Drehzahl zwischen 1.000 und 6.000 U/min um die Dorndrehachse (242) gedreht wird und der kombinierte Dorn (250) während des Sammelns des zweiten Abschnitts (254) der Polymerfasern mit einer Drehzahl zwischen 1.000 und 6.000 U/min um die Dorndrehachse (242) gedreht wird; oder
b) wobei der erste Dorn (200) um die Dornrotationsachse (242) mit einer Rotationsgeschwindigkeit zwischen 2.000 und 4.000 U/min während des Sammelns des ersten Abschnitts der Polymerfasern (240) gedreht wird und der kombinierte Dorn (250) um die Dornrotationsachse (242) mit einer Rotationsgeschwindigkeit zwischen 2.000 und 4.000 U/min während des Sammelns des zweiten Abschnitts (254) der Polymerfasern gedreht wird.

7. Verfahren nach Anspruch 6, bei dem ferner der rotierende kombinierte Dorn (250) während des Sammelns des zweiten Abschnitts (254) der Polymerfasern linear verschoben wird.

8. Verfahren nach Anspruch 4, wobei eine Oberfläche des zweiten Dorns (220) einen oder mehrere Blättchen ausbildende Abschnitte aufweist, von denen jeder eine Form aufweist, die einer zweiten Oberfläche eines entsprechenden von dem ein oder den mehreren Blättchen (320a, 320b) in dem entstehenden Ventil (300, 400) entspricht; wahlweise, wobei der mindestens eine erste Blättchen ausbildende Abschnitt (206a, 206b) des ersten Dorns (200) eine konkave Form aufweist und der mindestens eine Blättchen ausbildende Abschnitt des zweiten Dorns (220) eine konvexe Form (228a, 228b, 228c) aufweist.

9. Verfahren nach Anspruch 4, wobei der erste Teil der Polymerfasern (240) zwischen dem ersten Dorn (200) und dem zweiten Dorn (220) und auf dem ersten röhrenförmigen Formungsabschnitt (204) der Außenfläche (202) des ersten Dorns (200) angeordnet wird, wenn der zweite Dorn (220) in Bezug auf den ersten Dorn (200) angeordnet wird, um den kombinierten Dorn (250) auszubilden;
wahlweise, wobei der kombinierte Dorn (250) einen Spalt zwischen dem ersten Dorn (200) und dem zweiten Dorn (220) ausbildet, worin sich der Spalt von der Außenfläche des kombinierten Dorns (250) radial nach innen erstreckt;
wahlweise, wobei der erste Teil der Polymerfasern (240) innerhalb des Spaltes angeordnet wird und sich mindestens bis zu einer Grenze zwischen dem Spalt und dem ersten röhrenförmigen Formungsabschnitt (204) erstreckt, so dass, wenn der zweite Teil (254) der Polymerfasern auf dem kombinierten Dorn (250) gesammelt wird, der zweite Teil (254) der Polymerfasern mit dem ersten Teil der Polymerfasern (240) einstückig ausgebildet wird.

10. Verfahren nach Anspruch 4, wobei das Anordnen des zweiten Dorns (220) in Bezug auf den ersten Dorn (200) die Eingriffnahme von mindestens einem ersten Eingriffsabschnitt (208a, 208b, 208c) des ersten Dorns (200) mit einem entsprechenden mindestens einen zweiten Eingriffsabschnitt (228a) des zweiten Dorns (220) umfasst; wahlweise, wobei der mindestens eine erste Eingriffsabschnitt (208a, 208b, 208c) mindestens einen Vorsprung umfasst und der mindestens eine zweite Eingriffsabschnitt (228a) mindestens eine Aussparung umfasst, die zur Aufnahme des mindestens einen Vorsprungs (208a, 208b, 208c) ausgestaltet ist.

11. Verfahren nach Anspruch 4, welches ferner umfasst:
(a) Anordnen eines Stents (276) über dem zweiten Teil (254) der Polymerfasern, die auf der Außenfläche des kombinierten Dorns (250) gesammelt wurden; und
Sammeln eines dritten Abschnitts (280) der Polymerfasern auf einer Außenfläche des Stents (276), um die rohrförmige Wand (310, 410) mit dem darin eingebetteten Stent (276) auszubilden; oder
(b) Aussäen von Zellen auf die Polymerfasern der röhrenförmigen Wand (310, 410).

12. Verfahren nach Anspruch 4, worin die röhrenförmige Wand (310, 410) ein erstes Ende (301a) und ein zweites Ende (301b) aufweist, wobei das eine oder die mehreren Blättchen (320a, 320b) zwischen dem ersten Ende (301a) und dem zweiten Ende (301b) angeordnet wird, und wobei das Verfahren ferner umfasst:
Herausziehen des ersten Dorns (200) aus dem Inneren der röhrenförmigen Wand (310, 410) über das erste Ende (301a); und
Herausziehen des zweiten Dorns (220) aus dem Inneren der röhrenförmigen Wand (310, 410) über das zweite Ende (301b).

13. Verfahren nach Anspruch 4, worin jede gebildete Polymerfaser einen Durchmesser zwischen etwa 0,5 µm und etwa 1,5 µm aufweist oder worin ein durchschnittlicher Durchmesser der gebildeten Polymerfasern zwischen etwa 0,75 µm und etwa 1,25 µm liegt.

14. Verfahren nach Anspruch 4, worin der erste Dorn (200) mindestens zwei Blättchen ausbildende Abschnitte (206a, 206b) aufweist, wobei das Sammeln des ersten Abschnitts (270) der gebildeten Polymerfasern (240) auf der Außenfläche (202) des ersten Dorns (200) zumindest teilweise den ersten Abschnitt (270) der röhrenförmigen Wand (310, 410) und jedes von zwei oder mehr Blättchen (320a, 320b) ausbildet, die mit der Innenfläche des ersten Abschnitts (270) der röhrenförmigen Wand (310, 410) verbunden sind und einstückig damit vorliegen.

## Revendications

1. Valvule technique (300, 400) comprenant :
une paroi tubulaire (310, 410) comprenant des fibres polymères de dimensions micrométriques, sous-micrométriques ou nanométriques définissant une forme de la paroi tubulaire (310, 410), la paroi tubulaire (310, 410) ayant une surface interne, une première portion (270), et une deuxième portion (274) ;
un ou plusieurs feuillets (320a, 320b) s'étendant à partir, et formant partie intégrante, de la surface interne de la paroi tubulaire (310, 410), les un ou plusieurs feuillets (320a, 320b) comprenant chacun des fibres polymères de dimensions micrométriques, sous-micrométriques ou nanométriques définissant la forme du feuillet (320a, 320b) correspondant ;
dans laquelle les un ou plusieurs feuillets (320a, 320b) sont configurés pour un écoulement de fluide unidirectionnel à travers la première portion (270) de la paroi tubulaire (310, 410), au-delà des un ou plusieurs feuillets (320a, 320b), et dans la deuxième portion (272) de la paroi tubulaire (310, 410), et dans lequel la deuxième portion de la paroi tubulaire (272) inclut la totalité de la paroi tubulaire (310, 410) en aval des un ou plusieurs feuillets (320a, 320b) ;
dans laquelle, pour chacun des un ou plusieurs feuillets (320a, 320b), au moins certaines des fibres polymères de dimensions micrométriques, sous-micrométriques ou nanométriques (240) formant une partie du feuillet (320a, 320b) s'interpénètrent avec au moins certaines des fibres polymères de dimensions micrométriques, sous-micrométriques ou nanométriques formant une partie de la première portion (270) de la paroi tubulaire (310, 410) ; et
dans laquelle les fibres polymères de dimensions micrométriques, sous-micrométriques ou nanométriques de la paroi tubulaire (310, 410) et les fibres polymères de dimensions micrométriques, sous-micrométriques ou nanométriques (240) des un ou plusieurs feuillets (320a, 320b) sont configurées pour former un échafaudage de fibres polymères pour une interposition cellulaire dans la paroi tubulaire (310, 410) et les un ou plusieurs feuillets (320a, 320b).

2. Valvule technique (300, 400) selon la revendication 1, dans laquelle
(a) au moins certaines des fibres polymères de dimensions micrométriques, sous-micrométriques ou nanométriques des un ou plusieurs feuillets (320a, 320b) s'interpénètrent avec au moins certaines des fibres polymères de dimensions micrométriques, sous-micrométriques ou nanométriques de la surface interne de la paroi tubulaire (310, 410) ; ou
(b) les fibres polymères de dimensions micrométriques, sous-micrométriques ou nanométriques des un ou plusieurs feuillets (320a, 320b) s'interpénètrent avec les fibres polymères de dimensions micrométriques, sous-micrométriques ou nanométriques de la surface interne de la paroi tubulaire (310, 410) où les un ou plusieurs feuillets (320a, 320b) rencontrent la surface interne de la paroi tubulaire (310, 410) ; ou
(c) les feuillets (320a, 320b) forment aussi partie intégrante de la surface externe de la paroi tubulaire (310, 410) ; ou
(d) la paroi tubulaire (310, 410) comprend en outre un stent (276) intégré dans les fibres polymères de dimensions micrométriques, sous-micrométriques ou nanométriques ; ou
(e) les fibres polymères de dimensions micrométriques, sous-micrométriques ou nanométriques ont chacune un diamètre compris entre environ 0,5 µm et environ 1,5 µm.

3. Valvule technique (300, 400) selon la revendication 1, dans laquelle la valvule (300, 400) est formée par :
formation d'une première portion des fibres polymères par éjection ou projection d'un polymère à partir d'un réservoir (102) sur un premier mandrin (200) ;
collecte de la première portion de fibres polymères formées (240) sur une surface externe (202) du premier mandrin (200) pour au moins partiellement former la première portion (270) de la paroi tubulaire (310, 410) et les un ou plusieurs feuillets (320a, 320b) raccordés à, et formant partie intégrante de, une surface interne de la première portion (270) de la paroi tubulaire (310, 410), les fibres polymères formées incluant des fibres polymères de dimensions micrométriques, sous-micrométriques ou nanométriques, la surface externe (202) du premier mandrin incluant :
une première portion de formation tubulaire (204) ayant une forme correspondant à la surface interne de la première portion (270) de la paroi tubulaire (310, 410), et
une ou plusieurs portions de formation de feuillet (206a, 206b), ayant chacune une forme correspondant à une première surface (322a) de l'un correspondant des un ou plusieurs feuillets (320a, 320b) de la valvule résultante (300, 400) ;
le positionnement d'un second mandrin (220) par rapport au premier mandrin (200) ayant la première portion collectée de fibres polymères (240) sur celui-ci, le second mandrin (220) ayant une surface extérieure (222) incluant une deuxième portion de formation tubulaire (224) ayant une forme correspondant à une surface interne d'une deuxième portion (272) de la paroi tubulaire résultante (310, 410), le premier mandrin (200) et le second mandrin (220) formant conjointement un mandrin combiné (250) ;
la formation d'une deuxième portion (254) des fibres polymères par éjection ou projection du polymère à partir du réservoir (102) sur le mandrin combiné (250) ; et
la collecte de la deuxième portion (254) des fibres polymères sur une surface extérieure du mandrin combiné (250) formant ainsi, au moins, la deuxième portion (272) de la paroi tubulaire (310, 410).

4. Procédé de fabrication d'une valvule (300, 400) incluant une paroi tubulaire (310, 410) et un ou plusieurs feuillets (320a, 320b) formant partie intégrante et s'étendant à partir d'une surface interne de la paroi tubulaire (310, 410), le procédé comprenant :
la collecte d'une première portion de fibres polymères formées (240) sur une surface extérieure (202) d'un premier mandrin (200) pour former au moins partiellement une première portion (270) d'une paroi tubulaire (310, 410) et un ou plusieurs feuillets (320a, 320b) raccordés à, et formant partie intégrante de, une surface interne de la première portion (270) de la paroi tubulaire (310, 410), les fibres polymères formées incluant des fibres polymères de dimensions micrométriques, sous-micrométriques et/ou nanométriques, dans lequel, pour chacun des un ou plusieurs feuillets (320a, 320b), au moins certaines des fibres polymères de dimensions micrométriques, sous-micrométriques ou nanométriques formant une partie du feuillet (320a, 320b) s'interpénètrent avec au moins certaines des fibres polymères de dimensions micrométriques, sous-micrométriques ou nanométriques formant une partie de la première portion amont (270) de la paroi tubulaire (310, 410), la surface extérieure (202) du premier mandrin incluant :
une première portion de formation tubulaire (204) ayant une forme correspondant à la surface interne de la première portion (270) de la paroi tubulaire (310, 410), et
une ou plusieurs portions de formation de feuillet (206a, 206b), ayant chacune une forme correspondant à une première surface (322a) de l'un correspondant des un ou plusieurs feuillets (320a, 320b) de la valvule résultante (300, 400) ;
le positionnement d'un second mandrin (220) par rapport au premier mandrin (200) ayant la première portion collectée de fibres polymères (240) sur celle-ci, le second mandrin (220) ayant une surface externe (222) incluant une deuxième portion de formation tubulaire (224) ayant une forme correspondant à une surface interne d'une deuxième portion (272) de la paroi tubulaire résultante (310, 410), le premier mandrin (200) et le second mandrin (220) formant conjointement un mandrin combiné (250) ; et
la collecte d'une deuxième portion (254) des fibres polymères formées sur une surface externe du mandrin combiné (250) formant ainsi, au moins, la deuxième portion (272) de la paroi tubulaire (310, 410), dans lequel la deuxième portion (272) de la paroi tubulaire résultante est la portion de la paroi tubulaire résultante qui est en aval des un ou plusieurs feuillets (320a, 320b) durant l'écoulement unidirectionnel du fluide jusqu'à la valvule résultante (300, 400) ;
dans lequel les fibres polymères de dimensions micrométriques, sous-micrométriques ou nanométriques (310, 410) et les fibres polymères de dimensions micrométriques, sous-micrométriques ou nanométriques des un ou plusieurs feuillets (320a, 320b) sont configurées pour former un échafaudage de fibres polymère pour une interposition cellulaire dans la paroi tubulaire (310, 410) et les un ou plusieurs feuillets (320a, 320b).

5. Procédé selon la revendication 4, comprenant en outre :
la formation de la première portion des fibres polymères (240) par éjection ou projection d'un polymère à partir d'un réservoir (102) sur le premier mandrin (200) ; et
la formation de la deuxième portion (254) des fibres polymères par éjection ou projection du polymère à partir du réservoir (102) sur le mandrin combiné (250) ; facultativement dans lequel (a) les fibres polymères sont éjectées à partir du réservoir (102) et le réservoir (102) est en rotation à une vitesse comprise entre 20 000 et 60 000 tr/min ; ou (b) le réservoir (102) est en rotation à une vitesse comprise entre 25 000 et 35 000 tr/min,
facultativement, dans lequel le réservoir (102) est dépourvu de champ électrique haute tension durant la formation de la première portion des fibres polymères (240) et durant la formation de la deuxième portion des fibres polymères (254).

6. Procédé selon la revendication 5, dans lequel les fibres polymères sont éjectées à partir du réservoir (102) et le réservoir (102) est en rotation à une vitesse comprise entre 20 000 et 60 000 tr/min ;
dans lequel la collecte de la première portion des fibres polymères (240) comprend la mise en rotation du premier mandrin (200) autour d'un axe de rotation de mandrin (242) dans une trajectoire des fibres polymères éjectées ou projetées ; et
dans lequel la collecte de la deuxième portion (254) des fibres polymères comprend la mise en rotation du mandrin combiné (250) autour de l'axe de rotation de mandrin (242) dans une trajectoire des fibres polymères éjectées ou projetés ; facultativement,
a) dans lequel le premier mandrin (200) est en rotation autour de l'axe de rotation de mandrin (242) à une vitesse de rotation comprise entre 1 000 et 6 000 tr/min durant la collecte de la première portion des fibres polymères (240) et le mandrin combiné (250) est en rotation autour de l'axe de rotation de mandrin (242) à une vitesse de rotation comprise entre 1 000 et 6 000 tr/min durant la collecte de la deuxième portion (254) des fibres polymères ; ou
b) dans lequel le premier mandrin (200) est en rotation autour de l'axe de rotation de mandrin (242) à une vitesse de rotation comprise entre 2 000 et 4 000 tr/min durant la collecte de la première portion des fibres polymères (240) et le mandrin combiné (250) est en rotation autour de l'axe de rotation de mandrin (242) à une vitesse de rotation comprise entre 2 000 et 4 000 tr/min durant la collecte de la deuxième portion (254) des fibres polymères.

7. Procédé selon la revendication 6, comprenant en outre la translation linéaire du mandrin combiné (250) en rotation durant la collecte de la deuxième portion (254) des fibres polymères.

8. Procédé selon la revendication 4, dans lequel une surface du second mandrin (220) inclut une ou plusieurs portions de formation de feuillet, chacune ayant une forme correspondant à une seconde surface de l'un correspondant des un ou plusieurs feuillets (320a, 320b) dans la valvule résultante (300, 400) ; facultativement dans lequel l'au moins une première portion de formation de feuillet (206a, 206b) du premier mandrin (200) a une forme concave, et l'au moins une portion de formation de feuillet du second mandrin (220) a une forme convexe (228a, 228b, 228c).

9. Procédé selon la revendication 4, dans lequel la première portion des fibres polymères (240) est disposée entre le premier mandrin (200) et le second mandrin (220) et sur la première portion de formation tubulaire (204) de la surface externe (202) du premier mandrin (200) lorsque le second mandrin (220) est positionné par rapport au premier mandrin (200) pour former le mandrin combiné (250) ;
facultativement, dans lequel le mandrin combiné (250) forme une crevasse entre le premier mandrin (200) et le second mandrin (220) avec la crevasse s'étendant radialement vers l'intérieur à partir de la surface externe du mandrin combiné (250) ;
facultativement, dans lequel la première portion des fibres polymères (240) est disposée à l'intérieur de la crevasse et s'étend au moins jusqu'à une limite entre la crevasse et la première portion de formation tubulaire (204), de telle sorte que lorsque la deuxième portion (254) des fibres polymères est collectée sur le mandrin combiné (250) la deuxième portion (254) des fibres polymères s'intègre avec la première portion des fibres polymères (240).

10. Procédé selon la revendication 4, dans lequel le positionnement du second mandrin (220) par rapport au premier mandrin (200) comprend la mise en prise d'au moins une première portion de mise en prise (208a, 208b, 208c) du premier mandrin (200) avec au moins une seconde portion de mise en prise (228a) correspondante du second mandrin (220) ; facultativement dans lequel l'au moins une première portion de mise en prise (208a, 208b, 208c) comprend au moins une saillie, et l'au moins une seconde portion de mise en prise (228a) comprend au moins une cavité configurée pour recevoir l'au moins une saillie (208a, 208b, 208c).

11. Procédé selon la revendication 4, comprenant en outre :
(a) le positionnement d'un stent (276) par-dessus la deuxième portion (254) des fibres polymères collectées sur la surface externe du mandrin combiné (250) ; et
la collecte d'une troisième portion (280) des fibres polymères sur une surface externe du stent (276) pour former la paroi tubulaire (310, 410) avec le stent (276) intégré à l'intérieur de celle-ci ; ou
(b) l'ensemencement des cellules sur les fibres polymères de la paroi tubulaire (310, 410).

12. Procédé selon la revendication 4, dans lequel la paroi tubulaire (310, 410) inclut une première extrémité (301a) et une seconde extrémité (301b) avec les un ou plusieurs feuillets (320a, 320b) disposés entre la première extrémité (301a) et la seconde extrémité (301b), et dans lequel le procédé comprend en outre :
le retrait du premier mandrin (200) de l'intérieur de la paroi tubulaire (310, 410) via la première extrémité (301a) ; et
le retrait du second mandrin (220) de l'intérieur de la paroi tubulaire (310, 410) via la seconde extrémité (301b) .

13. Procédé selon la revendication 4, dans lequel chaque fibre polymère formée a un diamètre compris entre environ 0,5 µm et environ 1,5 µm, ou dans lequel le diamètre moyen des fibres polymères formées est compris entre environ 0,75 µm et environ 1,25 µm.

14. Procédé selon la revendication 4, dans lequel le premier mandrin (200) inclut au moins deux portions de formation de feuillet (206a, 206b), dans lequel la collecte de la première portion (270) des fibres polymères formées (240) sur la surface externe (202) du premier mandrin (200) forme au moins partiellement la première portion (270) de la paroi tubulaire (310, 410) et chacun de deux feuillets (320a, 320b) ou plus raccordé à, et formant partie intégrante de, la surface interne de la première portion (270) de la paroi tubulaire (310, 410) .
